# EUROPEAN PATENT APPLICATION

(11) **EP 3 406 254 A1**
(43) Date of publication of application: **28.11.2018**
(21) Application number: 17741111.3
(22) Date of filing: 20.01.2017
(51) Int. Cl.: A61K 33/44, A61K 47/04, A61K 47/50, A61P 39/06, A61P 7/00, A61P 7/02, A61P 43/00, A61P 1/16, A61P 1/00, A61P 13/12

(54) **APPLICATION OF FULLERENE/METAL-FULLERENE FOR PREPARING PHARMACEUTICAL PRODUCT**

(30) Priority: 21.01.2016 CN 201610041914; 11.08.2016 CN 201610656821
(71) Applicant: Beijing Fullcan Biotechnology Co., Ltd, Beijing 100089 (CN)
(72) Inventor: WANG, Chunru, Beijing 100089 (CN); ZHEN, Mingming, Beijing 100089 (CN); ZHANG, Ying, Beijing 100089 (CN); BAI, Chunli, Beijing 100089 (CN)
(74) Representative: LLR
(86) International application number: PCT/CN2017/071971
(87) International publication number: WO 2017/125080

(57) **Abstract**

Provided is an application of a fullerene/metal-fullerene micro/ nanomaterial for preparing a pharmaceutical product. The pharmaceutical product has at least one of the following properties:1) treats a bone marrow suppression; 2) treats at least one of the following conditions caused by the bone marrow suppression: white blood cell count reduction, platedlet count reduction, hemo-globin count reduction, and monocyte count reduction; 3) protects a bone marrow cell and/or a hematopoietic cell; 4) able to build up in a bone marrow; 5)protects the liver, spleen, and kidneys; 6) removes a free radical; and 7) improves the performance of an antioxidant system in an organism.

## Description

### CROSS REFERENCE

The present disclosrue claims the priority of the Chinese patent application having an application number of CN201610041914.1 and entitled "Fullerene Micro-nano Material with Effects of Prevention and/or Treatment on Myelosuppression and Use Thereof', which is filed to State Intellectual Property Office by Beijing Fullcan Biotech Co., Ltd. and the Institute of Chemistry of Chinese Academy of Sciences, and is incorporated herein by reference in its entirety.

The present disclosrue also claims the priority of the Chinese patent application having an application number of CN201610656821.X and entitled "Micro-nano Material for Preventing and/or Treating Myelosuppression and Use Thereof', which is filed to State Intellectual Property Office by the Institute of Chemistry of Chinese Academy of Sciences and Beijing Fullcan Biotech Co., Ltd., and is incorporated herein by reference in its entirety.

### FIELD OF THE DISCLOSRUE

The present disclosrue relates to the field of biological medicine, and more particularly, to a use of a fullerene micro-nano material and/or a metallofullerene micro-nano material in the preparation of a medicament for treating myelosuppression.

### BACKGROUND ART

Recent years have witnessed an increasingly growing incidence of malignant tumors, which seriously endangers people's health. Although there emerge various novel methods and means for treating cancers as a result of technological advancements, the therapies based on chemotherapeutic drugs and radiotherapeutic rays are still commonly used in the clinical treatment of malignant tumors. Currently, 60-70% of the patients suffering from cancers are required to be treated with chemotherapeutic drugs or radiotherapeutic rays. Generally speaking, "metastases" can be exterminated promptly if a patient is treated by proper radiotherapy or chemotherapy 2-4 weeks after his tumor is surgically removed. As such, tumor recurrence or metastasis can be effectively inhibited. However, most chemotherapeutic drugs and radiotherapeutic rays would impair normal cells with active proliferation in human bodies while killing cancer cells, thereby exhibiting strong side effects. Particularly, with respect to bone marrow cells, hematopoietic stem cells, etc., killing effects of chemotherapeutic drugs and radiotherapeutic rays on them would cause secondary harm to a patient. Myelosuppression caused by radiotherapy and chemotherapy would be initially manifested by leucopenia and platelet reduction, and in severe cases, both red blood cells and hemoglobin would be reduced, or such diseases as aplastic anemia and leukemia would even be induced. Therefore, whether the marrow hematopoiesis of a patient can be protected during radiotherapy and chemotherapy and whether myelosuppression caused thereby can be alleviated are the critical factors that dictate whether the patient can adhere to such therapies. Although many traditional chinese medicine and western medicine are proved to be effective in preventing and treating myelosuppression, uses thereof are limited as these medicaments not only exhibit poor absorptivity within the human gastrointestinal tract, but also tend to produce adverse effects thereon. Another effective therapy is one in which the recombinant human granulocyte-colony stimulating factor is utilized, however, this therapy could be expensive and defective, and thus can't be widely used at present. Therefore, the exploration for an ideal drug capable of attenuating myelosuppression toxicity caused by chemotherapeutic drugs and radiotherapeutic rays has important clinical significance in alleviating a patient's conditions and further treating tumors.

In addition to graphite, diamond and amorphous carbon, fullerene is also an allotrope of carbon. Such substances refer to cage structures composed of carbon atoms, including C60, C70, C76, C78, C82 and C84, etc. In addition, as the interior of its carbon cage is structured as a cavity, the fullerenes of which different atoms, ions or clusters may be embedded in the cavity, is referred to as metallofullerene, such as La@C60, where La is embedded in the cage structure of C60, and @ represents at, which vividly expresses the meaning of embedding.

The information disclosed in this Background section is merely for the purpose of enhancing understanding of the general context of the present disclosrue, and should not be taken as an acknowledgement or any form of suggestion that this information forms the prior art already known to those of ordinary skill in the art.

### SUMMARY OF THE DISCLOSRUE

It is an object of the present disclosure to provide a use of fullerene micro-nano material and/or metallofullerene micro-nano material in the preparation of a medicament for treating myelosuppression. And it is a further object of the present disclosure to provide a pharmaceutical composition and method for treating myelosuppression. The fullerene micro-nano material and/or metallofullerene micro-nano material of the present disclosure still possesses the features of fullerene molecules, such as high conjugacy, efficient scavenging of free radicals, etc, and may be enriched in bone marrow efficiently and readily. The fullerene micro-nano material and/or metallofullerene micro-nano material of the present disclosure has an excellent function of preventing and treating myelosuppression in bodies, which is able to effectively reduce toxic and side effects of radiotherapeutic rays and chemotherapeutic drugs on bone marrow and other organs, and has treatment effects on myelosuppression-induced leucopenia, platelet reduction, hemoglobin reduction and monocyte reduction. Besides, this material is safe and non-toxic, and is able to be metabolized out of organisms.

In order to achieve the above subjects, the present disclosure provides the following technical solution.

A use of fullerene micro-nano material and/or metallofullerene micro-nano material in the preparation of a medicament, wherein the medicament has at least one of the following properties 1)-7):
1) treatment on myelosuppression; 2) treatment on at least one of myelosuppression-induced leucopenia, platelet reduction, hemoglobin reduction and monocyte reduction; 3) protection on a bone marrow cell and/or a hematopoietic cell; 4) ability to be enriched in the bone marrow; 5) protection on liver tissue, spleen tissue and kidney tissue; 6) scavenging of free radicals; 7) improvement on the function of a antioxidation system of an organism.

The present disclosure also provides a method for treating myelosuppression, which comprises the step of administering an effective dosage of a fullerene micro-nano material and/or a metallofullerene micro-nano material into an organism in need of treatment on myelosuppression.

The present disclosure further provides a pharmaceutical composition for treating myelosuppression, which comprises a fullerene micro-nano material and/or metallofullerene micro-nano material, and further comprises at least one of pharmaceutically-acceptable carrier, pharmaceutically-acceptable diluent and pharmaceutically-acceptable exipient.

According to the above-mentioned use, method or pharmaceutical composition in another implementation, the fullerene micro-nano material and/or metallofullerene micro-nano material comprises at least one effective ingredient selected from the group consisting of: an oil-soluble fullerene micro-nano material, an oil-soluble metallofullerene micro-nano material, a composition of the oil-soluble fullerene micro-nano material and the oil-soluble metallofullerene micro-nano material, a water-soluble fullerene micro-nano material, a water-soluble metallofullerene micro-nano material, a composition of the water-soluble fullerene micro-nano material and the water-soluble metallofullerene micro-nano material, and pharmaceutically-acceptable esters of the above six ingredients or pharmaceutically-acceptable salts of the above six ingredients.

According to the above-mentioned uses, methods or pharmaceutical compositions in another implementation, the oil-soluble fullerene micro-nano material is obtained by subjecting a fullerene body material to oil solubility modification, and the oil-soluble metallofullerene micro-nano material is obtained by subjecting a metallofullerene body material to oil solubility modification.

According to the above-mentioned uses, methods or pharmaceutical compositions in another implementation, an effective approach for oil solubility modification is one in which the fullerene body material and/or metallofullerene body material is subjected to oil solubility modification under the action of covalent or non-covalent interactions, and specific oil solubility modification processes may be carried out based on the methods disclosed in the prior art. Alternatively, for example, the fullerene body material and/or metallofullerene body material is coated with edible oil to obtain the oil-soluble fullerene micro-nano material and/or oil-soluble metallofullerene micro-nano material, and the specific coating method may be ball milling or ultrasonication. Specifically, the fullerene body material and/or metallofullerene body material (both in a powder form) is mixed with the edible oil; then, a resulting mixture is subjected to ultrasonication or ball milling in a ball mill for a certain time; thereafter, the oil-soluble fullerene micro-nano material and/or oil-soluble metallofullerene micro-nano material is able to be prepared.

According to the above-mentioned uses, methods or pharmaceutical compositions in another implementation, after the step in which the mixture is subjected to ultrasonication or ball milling in the ball mill for a certain time, the specific coating method also comprises a step of purifying the prepared oil-soluble fullerene micro-nano material and/or oil-soluble metallofullerene micro-nano material: centrifugation is conducted to remove precipitates; then, a resulting supernatant is filtered to obtain the final product.

According to the above-mentioned uses, methods or pharmaceutical compositions in another implementation, each 0.05-500 mg of fullerene body material and/or metallofullerene body material is dispersed in each 1 ml of the edible oil in the mixture subjected to ultrasonication or ball milling, wherein the disclosure of this range should be regarded as the disclosure of all the values within the range, and alternatively, these values of fullerene body material and/or metallofullerene body material are in a range of 0.05-1 mg, 0.05-10 mg, 0.05-100 mg, etc.

According to the above-mentioned uses, methods or pharmaceutical compositions in another implementation, the mixture is subjected to ultrasonication or ball milling for a time ranging from 30 minutes to 15 hours.

According to the above-mentioned uses, methods or pharmaceutical compositions in another implementation, the fullerene body material and/or metallofullerene body material may have a concentration ranging from 1,200 ppm to 1,500 ppm (mg/kg) in the oil-soluble fullerene micro-nano material and/or oil-soluble metallofullerene micro-nano material, such as 1,500 ppm.

According to the above-mentioned uses, methods or pharmaceutical compositions in another implementation, the edible oil includes, without limitation, at least one of olive oil, linseed oil, sunflower seed oil, maize germ oil, corn oil and squalane.

According to the above-mentioned uses, methods or pharmaceutical compositions in another implementation, the oil-soluble fullerene micro-nano material and/or oil-soluble metallofullerene micro-nano material may specifically be at least one of edible oil coated gadolinium metallofullerene (e.g., edible oil coated Gd@C82), edible oil coated C60, edible oil coated C70 or edible oil coated C84, etc., wherein the "edible oil" refers to any one of olive oil, linseed oil, sunflower seed oil, maize germ oil, corn oil and squalane.

As can be learned from the foregoing description, the oil-soluble fullerene micro-nano material and/or oil-soluble metallofullerene micro-nano material is endowed with all the following properties: (1) the solubility in lipid obtained by coating the fullerene body material and/or metallofullerene body material with the edible oil, gives it greater ease to enter cells and reach visceral organs in an organism through blood circulation; (2) the carbon cage of the fullerene and/or metallofullerene in the material obtained by coating remains intact, thereby exhibiting excellent scavenging effects on free radicals.

According to the above-mentioned uses, methods or pharmaceutical compositions in another implementation, the water-soluble fullerene micro-nano material of the present disclosure is obtained by subjecting a fullerene body material to water solubility modification, and the water-soluble metallofullerene micro-nano material is obtained by subjecting a metallofullerene body material to water solubility modification.

According to the above-mentioned uses, methods or pharmaceutical compositions in another implementation, the water solubility modification may be conducted based on the method disclosed in the prior art. Alternatively, the water solubility modification may be conducted using any one of the following methods: method 1: the method in which covalent modification is conducted to introduce hydrophilic groups on the surface of the body material is generally realized by a solid-liquid or liquid-liquid reaction under the action of a base; specifically, a fullerene body material and/or a metallofullerene body material is mixed and reacted with hydrogen peroxide and a base (this base is sodium hydroxide or potassium hydroxide); thereafter, a water-soluble fullerene micro-nano material and/or a water-soluble metallofullerene micro-nano material is able to be prepared, wherein this micro-nano material is hydroxy-modified. If an amino-modified micro-nano material is desired, it can be obtained by replacing sodium hydroxide and/or potassium hydroxide in the above step with ammonia water. As for method 2, the method is one in which a water-soluble carrier cooperates with a fullerene body material and/or metallofullerene body material to form a corresponding water-soluble material by hydrophobic-hydrophobic interactions of noncovalent interaction.

According to the above-mentioned uses, methods or pharmaceutical compositions in another implementation, the step in which the fullerene body material and/or metallofullerene body material is mixed and reacted with hydrogen peroxide and the base is followed by a step of purification: washing with ethyl alcohol, and dialyzing.

According to the above-mentioned uses, methods or pharmaceutical compositions in another implementation, 50-300 mg of C60, C70 or Gd@C82 solid is weighed, and mixed and reacted with 5-30 ml of 20-40% hydrogen peroxide and 2-20 ml of base solution having a concentration of 1M-3M at a temperature of 50-100 °C until the corresponding C60, C70 or Gd@C82 solid is thoroughly dissolved. This description merely illustrates the relationship of addition proportions among various substances. In practical uses, specific reaction scales are not limited to such as 50-300 mg, 5-30 ml and 2-20 ml; rather, and may be scaled up.

According to the above-mentioned uses, methods or pharmaceutical compositions in another implementation, the water-soluble fullerene micro-nano material and/or water-soluble metallofullerene micro-nano material comprises at least one selected from the group consisting of: a fullerene with the outer surface of the carbon cage being modified with a hydrophilic group, a metallofullerene with the outer surface of the carbon cage being modified with a hydrophilic group, a fullerene carried by a water-soluble carrier and a metallofullerene carried by a water-soluble carrier. Here, the modified and carried fullerene and/or metallofullerene refers to the water-soluble fullerene micro-nano material and/or water-soluble metallofullerene micro-nano material obtained after the fullerene body material and/or metallofullerene body material is modified or carried.

According to the above-mentioned uses, methods or pharmaceutical composition in another implementation, the hydrophilic group comprises one or more of hydroxyl group, carboxyl group, sulfhydryl group and amino group.

According to the above-mentioned uses, methods or pharmaceutical compositions in another implementation, the water-soluble fullerene micro-nano material and/or the water-soluble metallofullerene micro-nano material may specifically be water-soluble hydroxylated gadolinium metallofullerene (GFNC, Gd@C82(OH)n) and water-soluble hydroxylated fullerene (e.g., water-soluble hydroxylated C60 (C60(OH)n) or water-soluble hydroxylated C70 (C70(OH)n)).

According to the above-mentioned uses, methods or pharmaceutical compositions in another implementation, the water-soluble carrier is a pharmaceutical carrier commonly used in the medical domain, which comprises at least one of lipidosome, polymeric micelle and protein. Alternatively, the polymeric micelle is poly(lactic-co-glycolic acid) polyethylene glycol (PEG-PLGA), polylysine or chitosan; the protein is albumin or transferrin.

According to the above-mentioned uses, methods or pharmaceutical compositions in another implementation, the water-soluble fullerene micro-nano material and/or water-soluble metallofullerene micro-nano material is endowed with all the following properties: (1) surface hydrophility, which enables the micro-nano material be injected intravenously into an organism, and be enriched in the bone marrow through blood circulation; (2) rigidity (i.e. less deformable), which enables the micro-nano material pass through gaps between endothelial cells of blood sinuses in the bone marrow through blood circulation, and into the bone marrow quickly by means of the difference between inner and outer pressure of the blood vessel.

According to the above-mentioned uses, methods or pharmaceutical composition in another implementation, the myelosuppression is induced by medical chemotherapy, a chemical toxicant or a medicament having a myelosuppressive side effect. Alternatively, the medical chemotherapy comprises using a medicament that is conventionally used in clinical practice at present and exhibits inhibition effects on bone marrow, which may be cyclophosphamide (CTX), adriamycin, cis-platinum or paclitaxel; alternatively, the chemical toxicants may be benzene or derivatives thereof (e.g., chlorambucil); alternatively, the medicament having a myelosuppressive side effect may be chloramphenicol, tetracycline, tapazole or indometacin.

According to the above-mentioned uses, methods or pharmaceutical compositions in another implementation, the myelosuppression is induced by tumor radiotherapy or a ray producing a myelosuppressive side effect. Alternatively, the ray utilized in radiotherapy may be the α-ray, β-ray, γ-ray or X-ray. Alternatively, the ray producing a myelosuppressive side effect may be radioactive rays to which a person engaged in related work is inevitably exposed.

According to the above-mentioned uses, methods or pharmaceutical compositions in another implementation, the fullerene body material comprises one or more of a cage structure having a general formula of C₂ₘ (wherein 30≤m≤60) and composed of carbon atoms, such as, C₆₀, C₇₀, C₈₄, etc.

According to the above-mentioned uses, methods or pharmaceutical compositions in another implementation, the metallofullerene body material comprises one or more of M@C₂ₙ, M₂@C₂ₙ, MA@C₂ₙ, M₃N@C₂ₙ, M₂C2@C₂ₙ, M₂S@C₂ₙ, M₂O@C₂ₙ and MₓA₃₋ₓN@C₂ₙ, wherein both M and A represent a metal element, and are selected from any one of a lanthanide metal element, Sc and Y, and wherein 30≤n≤60, and 0≤x≤3; such as Gd@C82. N represents nitrogen element, C represents carbon element and S represents sulfur element, and the lanthanide metal element comprises La, Ce, Pr, Nd, Pm, Sm, Eu, Gd, Tb, Dy, Ho, Er, Tm, Yb and Lu.

According to the above-mentioned uses, methods or pharmaceutical compositions in another implementation, the average grain size of the water-soluble fullerene micro-nano material and/or water-soluble metallofullerene micro-nano material is in a range of from 1 nm to 500 nm, and specifically, in a range of from 140 nm to 200 nm.

According to the above-mentioned uses, methods or pharmaceutical compositions in another implementation, the average grain size of the oil-soluble fullerene micro-nano material and/or the oil-soluble metallofullerene micro-nano material is in a range of from 0.5 nm to 50 nm, and specifically, in a range of from 0.5 nm to 10 nm, from 0.5 nm to 5 nm, from 0.5 nm to 2 nm or about 1nm.

According to the medicaments in the above-mentioned uses or the above-mentioned pharmaceutical compositions in another implementation, the medicament or the pharmaceutical composition may be a preparation of tablet, pill, pulvis, pastille, sachet, cachet, elixir, suspension, emulsion, solution, syrup, aerosol, ointment, soft and hard gelatin capsules, suppository, sterile injectable solution or sterile packaged powder injections. In the present disclosure, the preparation of the effective ingredient into a medicament or pharmaceutical compositions may be conducted using the methods known to those of ordinary skill in the art, so that the effective ingredient is able to be released quickly, released slowly or delayed to be released after the administration to a subject. For example, effective ingredients may be mixed with a carrier, diluted with a carrier or encapsulated in a carrier.

According to the medicaments in the above-mentioned uses or the above-mentioned pharmaceutical compositions in another implementation, some examples suitable for use as the carrier, the exipient and the diluent comprise lactose, dextrose, sucrose, sorbitol, mannitol, starch, resin, arabic gum, calcium phosphate, alginate, tragacanth, gelatin, calcium silicate, microcrystalline cellulose, polyvinylpyrrolidone, cellulose, water syrup, methylcellulose, methylparaben and propylparaben, talcum powder, magnesium stearate and liquid paraffin.

According to the medicaments in the above-mentioned uses or the above-mentioned pharmaceutical compositions in another implementation, the medicament or pharmaceutical composition may additionally comprise an auxiliary comprising lubricant, wetting agent, emulsifier, suspending agent, preservative, sweetener or flavoring agent.

According to the medicaments in the above-mentioned uses or the above-mentioned pharmaceutical compositions in another implementation, the water-soluble fullerene micro-nano material and/or the water-soluble metallofullerene micro-nano material has a concentration of from 0.1 mM to 10mM in the preparation; the oil-soluble fullerene micro-nano material and/or the oil-soluble metallofullerene micro-nano material has a concentration of from 300 ppm to 5,000ppm (mg/kg) in the preparation.

According to the above-mentioned methods in another implementation, the fullerene micro-nano material and/or metallofullerene micro-nano material may be used within 24 hours before the administration of chemotherapeutic drugs or the use of radiotherapeutic rays, moreover, may also be used during radiotherapy and chemotherapy, and may also be used continuously within 1-2 weeks after radiotherapy and chemotherapy are finished.

According to the above-mentioned methods in another implementation, the effective ingredient are administered at a dosage of from 1 mg/kg/d to 500 mg/kg/d, and alternatively, at a dosage of from 1 mg/kg/d to 100 mg/kg/d, from 1 mg/kg/d to 20 mg/kg/d or from 1 mg/kg/d to 10 mg/kg/d.

According to the above-mentioned methods in another implementation, the administration is achieved by means of injection or oral ingestion; alternatively, the manner of injection is intraperitoneal or intravenous injection through which the effective ingredient enters a human body and directly exert their effects through blood circulation, which dispenses with osmosis, requires small dosages of medicaments and exhibits high therapeutic effects; the administration may also be achieved by oral ingestion through which medicaments are filtered and absorbed by the digestive system, which produces less side effects and exhibits remarkable therapeutic effects.

According to the above-mentioned methods in another implementation, the organism is a human or an animal, and the animal may be a mammal, such as, a mouse, a guinea pig, a rat, a dog, a rabbit, a monkey, etc.

The terms "treatment", "treating" or "treated "as used herein comprises its commonly accepted meanings, including restraining, preventing, inhibiting, improving, alleviating arresting or reversing the development of resultant symptoms or the development of expected lesions. Based on this, the present disclosure encompasses both therapeutic and preventative administration.

The terms "effective dosage" or "effective amount" as used herein refers to the amount of effective ingredient of fullerene micro-nano material and/or metallofullerene micro-nano material administered to an organism through the method of the present disclosure that is sufficient to effectively deliver for preventing myelosuppression. For patients suffering from mild myelosuppression, the medicament may be administered in a single dose, and for patients suffering from severe myelosuppression, the drug may be administered in multiple doses. After the administration of a single dose or multiple doses, therapeutic effects on myelosuppression are remarkable.

The term "effective ingredient" as used herein refers to at least one of a water-soluble fullerene micro-nano material, a water-soluble metallofullerene micro-nano material, a composition of the water-soluble fullerene micro-nano material and water-soluble metallofullerene micro-nano material, a oil-soluble fullerene micro-nano material, a oil-soluble metallofullerene micro-nano material, a composition of the oil-soluble fullerene micro-nano material and the oil-soluble metallofullerene micro-nano material, a pharmaceutically-acceptable ester and a pharmaceutically-acceptable salt of the above 6 ingredients.

The term "fullerene body material" as used herein refers to a fullerene that has not been carried out oil solubility modification or water solubility modification, namely the fullerene per se.

The term "metallofullerene body material" as used herein refers to a metallofullerene that has not been carried out oil solubility modification or water solubility modification, namely the metallofullerene per se.

In order to facilitate metering, all the quantitative limitations such as specific contents and concentrations for the water-soluble fullerene micro-nano material, the water-soluble metallofullerene micro-nano material, the oil-soluble fullerene micro-nano material and the oil-soluble metallofullerene micro-nano material in the present disclosure are measured based on specific contents, concentrations and the like of corresponding fullerene body material or metallofullerene body material. For example, the administered dosage of effective ingredient that ranges from 1 mg/kg/d to 500 mg/kg/d means that in the effective ingredient to be administered to every 1 kg of mice per day, the corresponding amount of carbon cages of fullerene body material or metallofullerene body material is 1-500 mg.

As compared with the prior art, the present disclosure has the following beneficial effects:
1. The fullerene micro-nano material and/or metallofullerene micro-nano material in the present disclosure still possesses the features of fullerene molecules, such as high conjugacy, efficient scavenging of free radicals, etc.
2. The fullerene micro-nano material and/or metallofullerene micro-nano material in the present disclosure is endowed with special physical and chemical properties, such that it can pass through nano-gaps between endothelial cells of blood sinuses in the bone marrow through blood circulation, and be enriched in visceral organs and the bone marrow efficiently and readily by means of the difference between inner and outer pressure of the blood vessel. The content of fullerene micro-nano material and/or metallofullerene micro-nano material in per unit mass of the bone marrow is more than 12 times of that in sclerotin. Moreover, the staying time of fullerene micro-nano material and/or metallofullerene micro-nano material in the bone marrow is obviously longer than that in other organs, indicating that it has excellent bone marrow-targeting ability.
3. The fullerene micro-nano material and/or metallofullerene micro-nano material in the present disclosure is able to scavenge free radicals efficiently, has an excellent function of preventing and treating myelosuppression in bodies, is able to effectively reduce toxic and side effects of radiotherapeutic rays and chemotherapeutic medicaments on the bone marrow and other organs, and has treatment effects on myelosuppression-induced leucopenia, platelet reduction, hemoglobin reduction and monocyte reduction. Moreover, the fullerene micro-nano material and/or metallofullerene micro-nano material is still obvious in protection effects after multiple treatment courses, and would not affect the therapeutic effects of radiotherapy and chemotherapeutic medicaments on tumors. Therefore, the fullerene micro-nano material and/or metallofullerene micro-nano material in the present disclosure is able to prevent and treat myelosuppression induced by chemotherapeutic medicaments, radiotherapeutic rays and other factors, and thus can be used to prepare medicaments for treating myelosuppression.
4. The fullerene micro-nano material and/or metallofullerene micro-nano material in the present disclosure can be prepared rapidly in a simple manner, exhibits no obvious cytotoxic effects on living organisms, bone marrow cells and normal cells, and is safe and non-toxic; moreover, this material can be metabolized out of organisms, and exhibit excellent biocompatibility.
5. The fullerene micro-nano material and/or metallofullerene micro-nano material in the present disclosure is also able to prevent the liver, kidney, spleen and other organs from being damaged.

### DESCRIPTION OF THE DRAWINGS

Figures 1A-1D illustrate blood routine indexes of mice in different groups in accordance with Embodiment 2 of the present disclosure.
Figure 2 is a curve illustrating weight changes of mice in different groups in accordance with Embodiment 2 of the present disclosure.
Figures 3A-3D illustrate enzyme indexes of mice in different groups in accordance with Embodiment 2 of the present disclosure.
Figures 4A-4D illustrate blood routine indexes of mice in different groups in accordance with Embodiment 3 of the present disclosure.
Figure 5 is a curve illustrating weight changes of mice in different groups in accordance with embodiment 3 of the present disclosure.
Figures 6A-6D illustrate enzyme indexes of mice in different groups in accordance with Embodiment 3 of the present disclosure.
Figures 7A-7D illustrate blood biochemical indexes of mice in different groups in accordance with Embodiment 3 of the present disclosure.
Figure 8 is a diagram illustrating the grain size distribution of water-soluble hydroxylated gadolinium metallofullerene prepared in Embodiment 4.
Figure 9 is a diagram illustrating the metabolic profile of water-soluble hydroxylated gadolinium metallofullerene within mice, as determined using ICP-MS in Embodiment 5.
Figure 10 is a diagram illustrating the metabolic profile of water-soluble hydroxylated gadolinium metallofullerene within mice, as determined using the ¹³¹I labeling method in Embodiment 6.
Figures 11A-F are electronic probe spectrograms illustrating water-soluble hydroxylated gadolinium metallofullerene samples in the bone marrow of mice at different time points in accordance with Embodiment 7.
Figure 12 illustrates coefficients of visceral organs for mice in a water-soluble hydroxylated gadolinium metallofullerene-injected group and a control group in accordance with Embodiment 8, wherein these coefficients are determined 30 days after injection.
Figure 13 illustrates weight changes of mice in the water-soluble hydroxylated gadolinium metallofullerene-injected group and the control group in accordance with Embodiment 8.
Figure 14 illustrates blood biochemical tests for mice in the water-soluble hydroxylated gadolinium metallofullerene-injected group in accordance with Embodiment 8, wherein test values all are determined 30 days after injection.
Figure 15 illustrates ESR results of free radical-scavenging experiment by GFNC in-vitro in accordance with Embodiment 9, wherein the solid line represents a control group; and the dotted line represents an experimental group.
Figure 16 illustrates experimental results of free radical scavenging by GFNC on a cellular level in accordance with Embodiment 10.
Figure 17 illustrates blood routine indexes of mice in different groups in accordance with Embodiment 11.
Figure 18 is a curve illustrating changes in tumor sizes of mice in different groups in accordance with Embodiment 12.
Figure 19 is a curve illustrating weight changes of mice in different groups in accordance with Embodiment 12.
Figure 20 illustrates blood routine indexes of mice in different groups in accordance with Embodiment 13.
Figure 21 is a curve illustrating changes in tumor sizes of mice in different groups in accordance with Embodiment 14.
Figure 22 is a curve illustrating weight changes of mice in different groups in accordance with embodiment 14.
Figure 23 illustrates blood biochemical indexes of mice in different groups in accordance with Embodiment 14.
Figure 24 is a section picture illustrating pathological sections of tissues and organs of mice in different groups after H&E staining in accordance with Embodiment 14.
Figure 25 is a diagram illustrating the grain size distribution of water-soluble hydroxylated hollow fullerene prepared in Embodiment 15.
Figure 26 illustrates coefficients of visceral organs for mice in a water-soluble hydroxylated hollow fullerene-injected group and a control group in accordance with Embodiment 16, wherein these coefficients are determined 30 days after injection.
Figure 27 illustrates weight changes of mice in the water-soluble hydroxylated hollow fullerene-injected group and the control group in accordance with Embodiment 16.
Figure 28 illustrates blood biochemical tests for mice in the water-soluble hydroxylated hollow fullerene-injected group in accordance with Embodiment 16, wherein test values are all determined 30 days after injection.
Figure 29 illustrates ESR results of an experiment in which free radicals are scavenged in vitro by the water-soluble hollow fullerene using according to Embodiment 17, wherein the solid line represents a control group; and the dotted line represents an experimental group.
Figure 30 illustrates results of an experiment in which free radicals are scavenged by a water-soluble derivative of C60 on a cellular level in accordance with Embodiment 18.
Figure 31 illustrates blood routine indexes of mice in different groups employing a water-soluble derivative of C60 in accordance with Embodiment 19.
Figure 32 illustrates blood routine indexes of mice in different groups employing a water-soluble derivative of C60 in accordance with Embodiment 20.

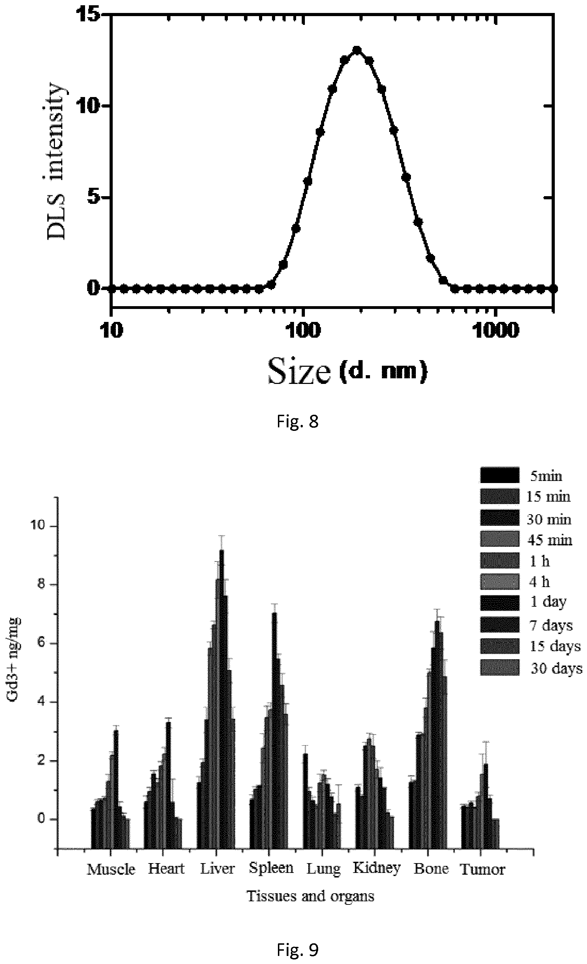

In the present disclosure, legends illustrated in the histograms of Figures 9, 10, 17, 20 and 32 in a top-to-bottom order correspond to the histograms arranged in a left-to-right order.

### DESCRIPTION OF THE PREFERRED EMBODIMENT

The present disclosure will now be described below through specific embodiments and in conjunction with the accompanying drawings, but not limited to the following embodiments.

Fullerene solid powders C60 and C70 as well as metallofullerene solid powder Gd@C82, which are used in the embodiments of the present disclosure, are purchased from Xiamen Funano New Material Technology Company LTD, and have a purity of 99%; the cyclophosphamide (CTX) used herein is purchased from Sigma-Aldrich Company, with the article number being C0768; the X-ray therapeutic instrument used herein is the same as that employed by the Department of Radiotherapy of Beijing Chaoyang Hospital. If not specified otherwise, the materials, reagents and instruments as used herein all are available from commercial sources, and other experimental methods all are conventional ones.

### Embodiment 1 Preparation of oil-coated hollow fullerene and/or oil-coated metallofullerene

1) Fullerene solid powder and/or metallofullerene solid powder, and a edible oil such as olive oil, are mixed together according to different proportions, and are then subjected to ball milling in a ball mill for 6-10 hours;
2) After ball milling is completed, centrifugation is carried out repeatedly in a centrifuge at a rotation speed of 10,000 rpm to remove the fullerene solid powder and/or metallofullerene solid powder that has not been successfully coated, until no solid powder is centrifuged down; thereafter, the oil-coated hollow fullerene and/or oil-coated metallofullerene is obtained.
3) The specific content of the successfully coated hollow fullerene solid powder and/or metallofullerene solid powder in the obtained oil-coated hollow fullerene and/or oil-coated metallofullerene is determined using an ultraviolet-visible spectrometer.

In a specific embodiment of the above-mentioned method, 100 mg of hollow fullerene C60 solid powder and 100 ml of olive oil are mixed together and are then subjected to ball milling for 6 hours; thereafter, after the hollow fullerene C60 solid powder that has not been successfully coated is removed by repeated centrifugation at a rotation speed of 10,000 rpm, an olive oil-coated hollow fullerene (hereinafter referred to as Olive-C60) is obtained. It is detected that the content of the hollow fullerene C60 solid powder in Olive-C60 is 1,500 ppm (mg/kg), i.e. that there is 1,500 mg of hollow fullerene C60 solid powder in every kilogram of Olive-C60.

### Embodiment 2 Effects of Olive-C60 on myelosuppression and relevant enzyme activities in the first course on an intravital level

Animal models: ICR mice aged 4-5 weeks were selected and assigned randomly into 5 groups with 6 mice per group. These groups were, respectively, a cyclophosphamide (CTX)+Olive-C60 intragastric administration experimental group, a CTX+Olive-C60 intraperitoneal administration experimental group, a CTX+Olive intragastric administration experimental group, a CTX experimental group and a blank control group.

Cyclophosphamide (CTX)+Olive-C60 intragastric administration experimental group: Mice were injected intraperitoneally with a CTX solution at a dosage of 60 mg/kg of body weight of mice; an Olive-C60 solution was administered intragastrically at a dosage of 100 µL (1,500 ppm).

Cyclophosphamide (CTX)+Olive-C60 intraperitoneal administration experimental group: Mice were injected intraperitoneally with a CTX solution at a dosage of 60 mg/kg of body weight of mice; an Olive-C60 solution was administered intragastrically at a dosage of 100 µL (1,500 ppm).

Cyclophosphamide (CTX)+Olive intragastric administration experimental group: Mice were injected intraperitoneally with a CTX solution at a dosage of 60 mg/kg of body weight of mice; a pure olive oil was administered intragastrically at a dosage of 100 µL.

Cyclophosphamide (CTX) experimental group: Mice were injected intraperitoneally with a CTX solution at a dosage of 60 mg/kg of body weight of mice; 100 µL of normal saline was administered intragastrically so as to eliminate the difference influenced by intragastric stimulation on mice.

Blank control group: The CTX solution injected in the experimental group was replaced with equal volumes of normal saline, and normal saline of the volume equal to 100µL was administered intragastrically or intraperitoneally to replace Olive-C60 or olive oil.

After ICR mice aged 4-5 weeks were bred in a laboratory, CTX solutions were injected on the seventh day when their conditions were stable, which was marked as the first day of the experiment. Intraperitoneal injection of CTX was conducted once a day for 5 consecutive days; meanwhile, Olive-C60 or Olive or the normal saline was administered intragastrically or intraperitoneally. 5 days later, no injection was made, and only observation or sampling was conducted.

Effects of Olive-C60 on myelosuppression in the first course on an intravital level: blood (13 µl) was taken from orbits of mice on the 4th day, the 7th day, the 10th day and the 17th day respectively, and was placed in a 3 ml centrifuge tube, and the blood routine examination was conducted using a blood cell automatic analyzer, wherein the main indexes related to myelosuppression are white blood cell counts (WBC), platelet counts (PLT), red blood cell counts (RBC) and hemoglobin determination (HGB). Meanwhile, changes in the weight of mice were monitored.

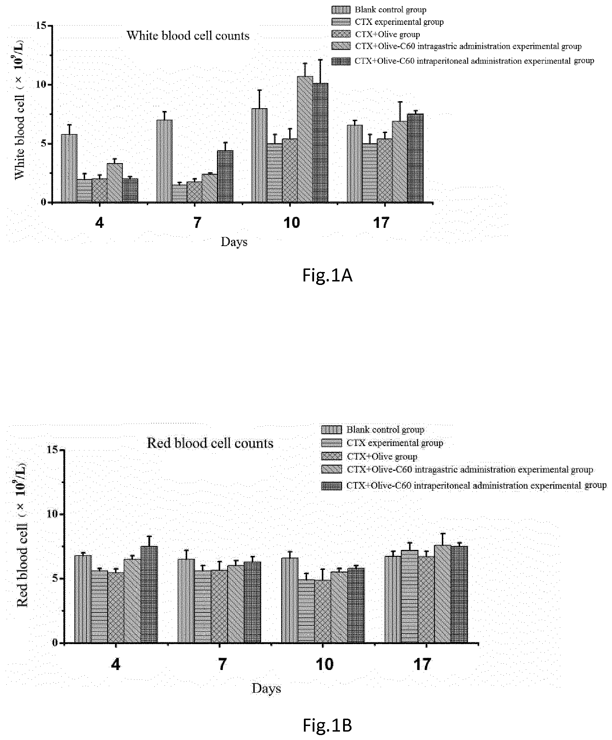

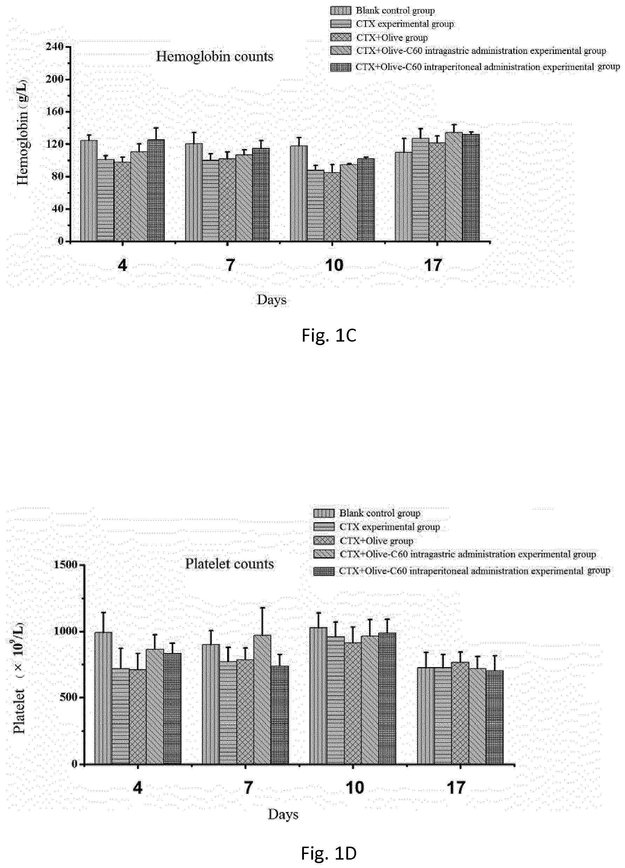

Corresponding detection results are as shown in Figures 1A-1D. It can be learned from Figures 1A-1D that as compared with the blank control group, the cyclophosphamide (CTX) experimental group exhibits the following effects: the myelosuppression-related indexes of mice, namely white blood cells, red blood cells, platelets and hemoglobin, are subjected to varying degrees of reduction, wherein the reduction in white blood cells is the most remarkable, which indicates that the bone marrow of mice is damaged, and significant abnormalities are also observed in related indexes; moreover, as for mice in the CTX+Olive-C60 experimental group, whether administration is conduced intraperitoneally or intragastrically, owing to the free radical-scavenging effects and protective effects of C60, the level of white blood cell counts is greatly improved as compared with that of the mice in the cyclophosphamide (CTX) experimental group, and values of red blood cells, platelets and hemoglobin are also closer to those of the blank control group. Moreover, as time progresses, related indexes are getting closer to those of normal mice. As for weight monitoring, in the initial stage of CTX injection, mice of different groups undergo weight loss due to the toxic and side effects of CTX; however, after termination of CTX injection, mice in the CTX+Olive-C60 experimental group regain weight at a faster rate than those in the CTX experiment group, and their weight levels are also closer to those of normal mice.

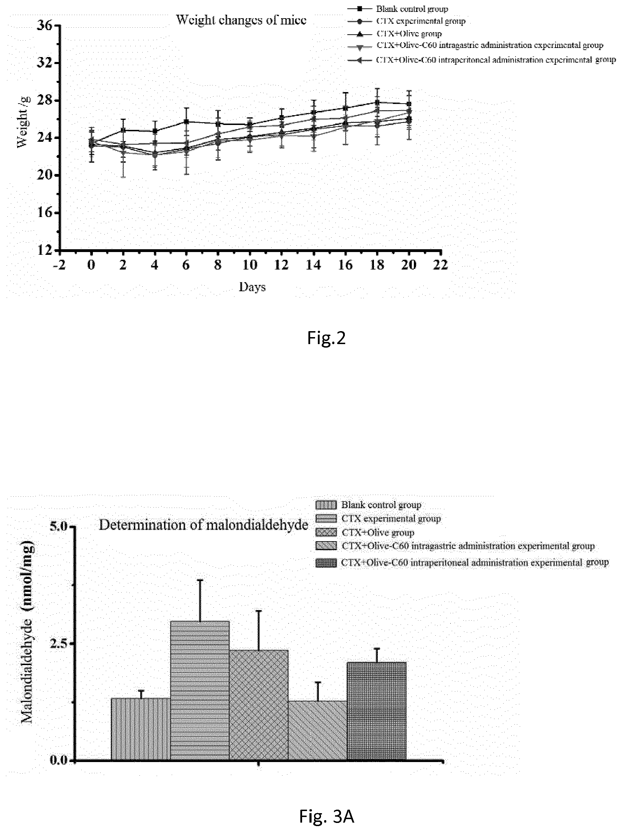

As indicated by the above data, Olive-C60 has obviously protective effects on mouse myelosuppression caused by chemotherapeutic medicament CTX, and protective effects of different administration manners all are remarkable. Meanwhile, as can be seen from Figure 2, in the later stage of the experiment, the weight of the mice in the cyclophosphamide experiment group is lower than that of the mice in any other groups, which also indicates the protective and improvement effects of Olive-C60 on the overall survival quality of mice.

Effects of Olive-C60 on relevant enzyme activities in the first course on an intravital level: On the 18th day of the experiment, whole blood was taken from orbits, serums were obtained by centrifugation, and different relevant enzyme activities were tested.

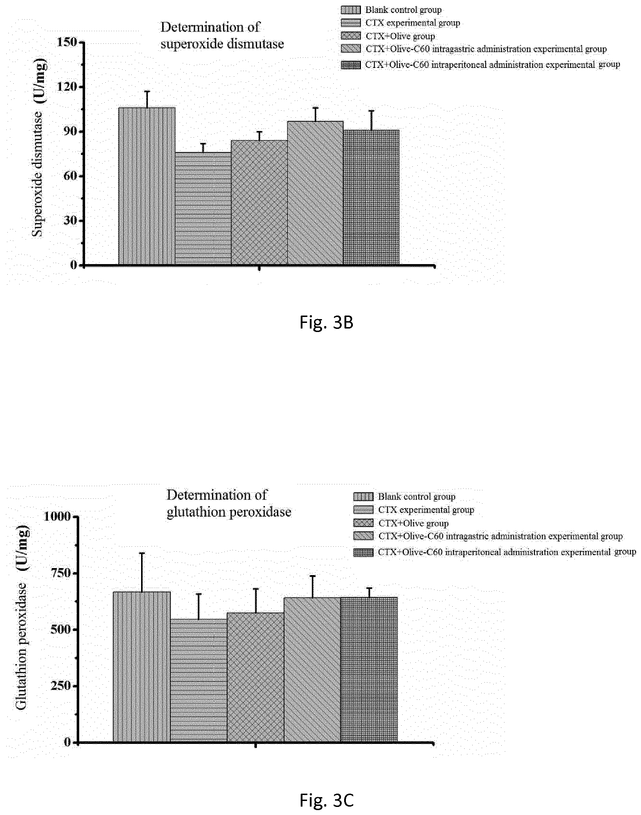

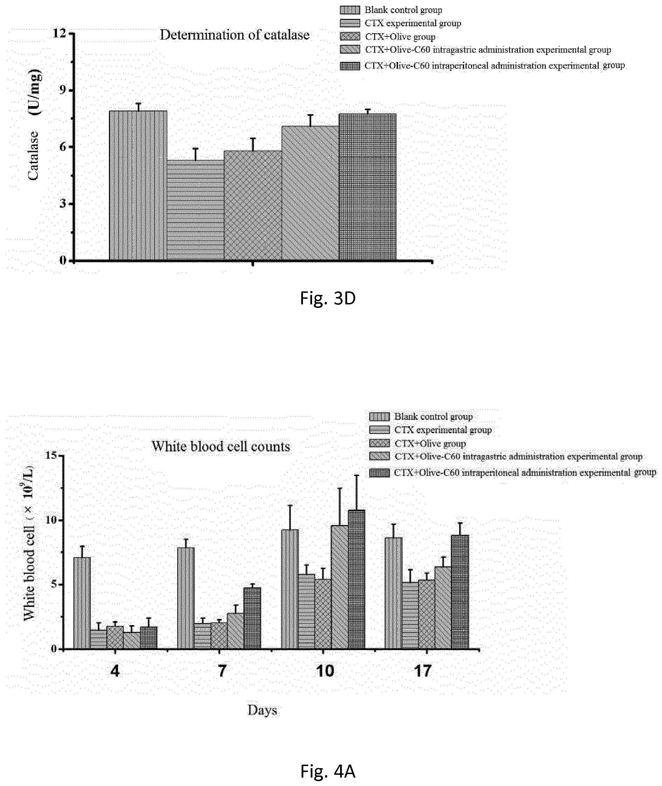

Corresponding detection results are as shown in Figures 3A-3D. As can be learned from Figures 3A-3D, in the treatment process, activities of the antioxidation-related enzymes (SOD, CAT and GPx) in the mice injected with olive oil-C60 are all higher than those of the enzymes in the mice in the CTX experimental group injected only with olive oil, and are also greatly higher than those of the enzymes in the mice dosed merely with cyclophosphamide. As peroxides within an organism are mainly removed by means of oxidation of the antioxidation system per se, this antioxidation system plays an extremely important role in improving the immunity of the organism and resisting against oxidative damage. Moreover, reduction in the activity of antioxidation enzymes would aggravate the damage to the organism. Therefore, the protection on the antioxidation system of the organism is of great significance to alleviating damage caused by chemotherapy. In this embodiment, it is obvious that the intake of Olive-C60 has obviously protective effects on the antioxidation system of the organism, thereby avoiding additional damage caused by chemotherapy. Moreover, MDA is generated as a result of lipid oxidation caused by free radicals, so the level of MDA in a body is a reflection of the level of free radicals in the body, and the increase in the level of MDA indicates that excessive free radicals which fail to be scavenged in time are present in the body, and that the damage to the cell membrane system is exacerbated. In this embodiment, by reducing the levels of such free radicals as hydroxyl radicals, Olive-C60 is able to alleviate the system damage of the cell membrane of the organism, and protect the organism from secondary harm caused by such chemotherapeutic medicaments as cyclophosphamide.

### Embodiment 3 Effects of Olive-C60 on myelosuppression, relevant enzyme activities and blood biochemical indexes in the second course on an intravital level

Animal models: ICR mice aged 4-5 weeks were selected and assigned randomly into 5 groups with 6 mice per group. These groups were, respectively, a cyclophosphamide (CTX)+Olive-C60 intragastric administration experimental group, a CTX+Olive-C60 intraperitoneal administration experimental group, a CTX+Olive intragastric administration experimental group, a CTX experimental group and a blank control group.

Cyclophosphamide (CTX)+Olive-C60 intragastric administration experimental group: Mice were injected intraperitoneally with a CTX solution at a dosage of 60 mg/kg of body weight of mice; an Olive-C60 solution was administered intragastrically at a dosage of 100 µL (1,500 ppm).

Cyclophosphamide (CTX)+Olive-C60 intraperitoneal administration experimental group: Mice were injected intraperitoneally with a CTX solution at a dosage of 60 mg/kg of body weight of mice; an Olive-C60 solution was administered intraperitoneally at a dosage of 100 µL (1,500 ppm).

Cyclophosphamide (CTX)+Olive intragastric administration experimental group: Mice were injected intraperitoneally with a CTX solution at a dosage of 60 mg/kg of body weight of mice; a pure olive oil was administered intragastrically at a dosage of 100 µL.

Cyclophosphamide (CTX) experimental group: Mice were injected intraperitoneally with a CTX solution at a dosage of 60 mg/kg of body weight of mice. 100 µL of normal saline was administered intragastrically so as to eliminate the difference influenced by intragastric stimulation on mice.

Blank control group: The CTX solution injected in the experimental group was replaced with equal volumes of normal saline, and normal saline of the volume equal to 100µL was administered intragastrically or intraperitoneally to replace Olive-C60 or olive oil.

The second course started 20 days after the day when the first course was initiated and mice were basically recovered. In other words, the 21st day of the first course was marked as the first day of the second course. Thereafter, intraperitoneal injection of CTX was conducted once a day for 5 consecutive days; and Olive-C60 or olive was administered intragastrically or intraperitoneally. 5 days later, no drug was given; and only observation or sampling was conducted.

Effects of Olive-C60 on myelosuppression in the second course on an intravital level: blood (13 µl) was taken from orbits of mice on the 4th day, the 7th day, the 10th day and the 17th day of the second course respectively, and was placed in a 3 ml centrifuge tube, and the blood routine examination was conducted using a blood cell automatic analyzer, wherein the main indexes related to myelosuppression are white blood cell counts (WBC), platelet counts (PLT), red blood cell counts (RBC) and hemoglobin determination (HGB). Meanwhile, changes in the weight of mice were monitored.

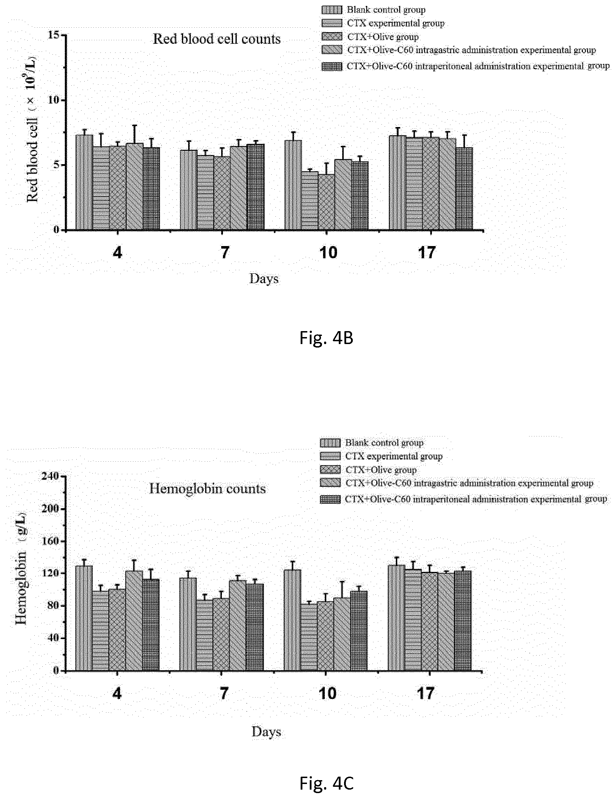

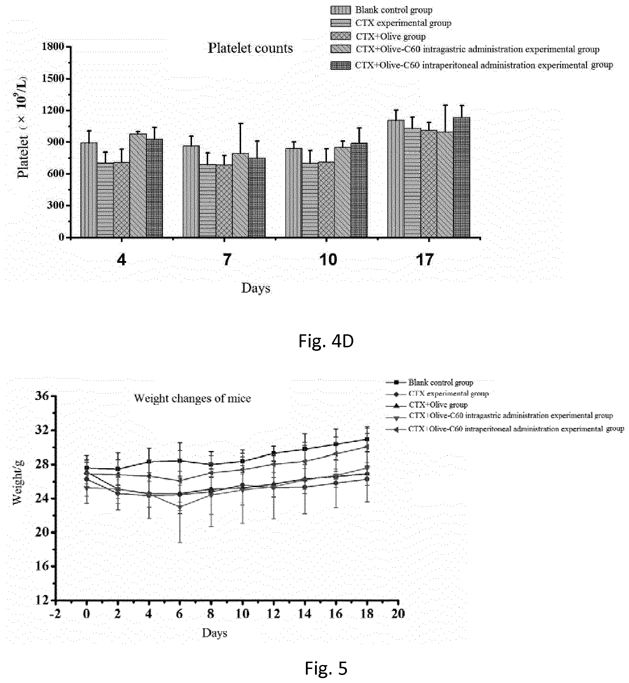

Corresponding detection results are as shown in Figures 4A-4D. It can be learned from Figures 4A-4D that during the process of the second course, as compared with the blank control group, the mice in the cyclophosphamide (CTX) experimental group exhibits the following myelosuppression-related indexes: white blood cells, red blood cells, platelets and hemoglobin, are subjected to varying degrees of reduction, wherein the reduction in white blood cells is the most remarkable, which indicates that the bone marrow of mice is subjected to secondary damage, and significant abnormalities are also observed in related indexes; moreover, as for mice in the CTX+Olive-C60 experimental group, whether administration is conduced intraperitoneally or intragastrically, owing to the free radical-scavenging effects and protective effects of C60, the level of white blood cell counts is greatly improved as compared with that of the mice in the cyclophosphamide (CTX) experimental group, and values of red blood cells, platelets and hemoglobin are also closer to those of the blank control group. Moreover, as time progresses, related indexes are getting closer to those of normal mice. On the 17th day, white blood cells of the mice in the Olive-C60 administration experiment groups are almost recovered to their normal levels, while those of the mice in the CTX experiment group are still below their normal levels. Meanwhile, as can be learned from the detection on the weight of the mice, the weight of the mice in the Olive-C60 administration experiment groups is closer to that of the mice in the blank control group, while the weight of the mice in the CTX experiment group differs greatly from that of the mice in other groups. The above results all indicate that Olive-C60 has obviously protective effects on mouse myelosuppression caused by CTX during the multiple-course administration. As such, the hematopoietic function of mice is able to recover to normal level more rapidly, and protective effects of different administration manners are all remarkable. Meanwhile, as can be seen from Figure 5, in the later stage of the experiment, the weight of the mice in the cyclophosphamide experiment group is lower than that of the mice in any other groups, which also indicates the protective and improvement effects of Olive-C60 on the overall survival quality of mice.

Effects of Olive-C60 on relevant enzyme activities in the second course on an intravital level: On the 18th day of the second course of the experiment, whole blood was taken from orbits, serums were obtained by centrifugation, and different relevant enzyme activities were tested.

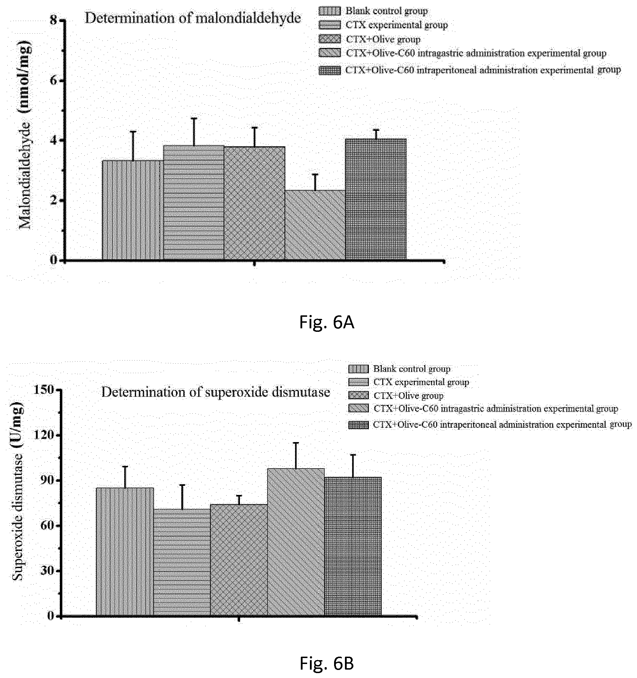

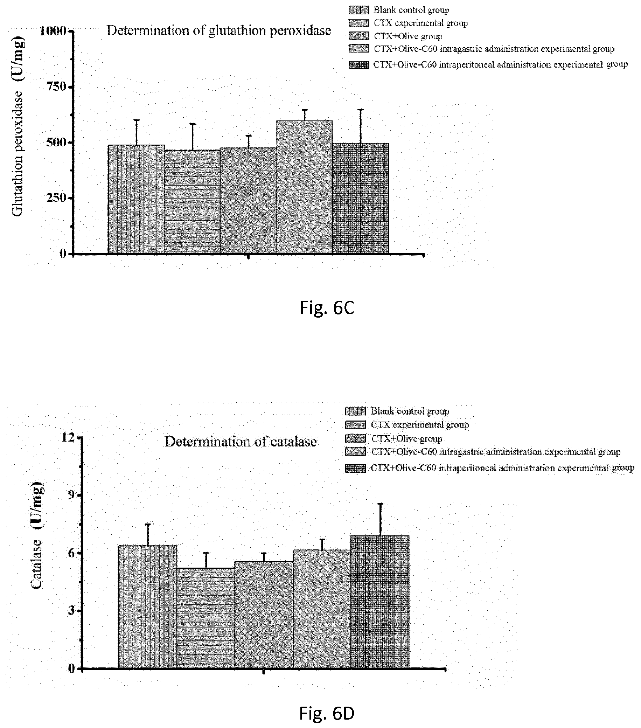

Corresponding detection results are as shown in Figures 6A-6D. As can be learned from Figures 6A-6D, after the multiple-course treatment process is completed, activities of the antioxidation-related enzymes (SOD, CAT and GPx) in the mice injected with Olive -C60 are all slightly higher than those of the enzymes in the mice in the experimental group injected only with olive oil, and are also higher than those of the enzymes in the mice dosed with cyclophosphamide. As described above, as peroxides within an organism are mainly removed by means of oxidation of the antioxidation system per se, this antioxidation system plays an extremely important role in improving the immunity of the organism and resisting against oxidative damage. Moreover, reduction in the activity of antioxidation enzymes would aggravate the damage to the organism. Therefore, the protection on the antioxidation system of the organism is of great significance to alleviating damage caused by chemotherapy. In this embodiment, it is obvious that the intake of Olive-C60 has obviously protective effects on the antioxidation system of the organism, thereby avoiding additional damage caused by chemotherapy. Moreover, MDA is generated as a result of lipid oxidation caused by free radicals, so the level of MDA in a body is a reflection of the level of free radicals in the body, and the increase in the level of MDA indicates that excessive free radicals which fail to be scavenged in time are present in the body, and that the damage to the cell membrane system is exacerbated. After multiple courses, the level of MDA of mice in the cyclophosphamide experiment group is higher than that of mice in the blank control group, while the level of MDA of the mice receiving Olive-C60 intragastricly is even lower than that of the mice in the blank control group, indicating that the Olive-C60 has remarkable scavenging effects on free radicals. In this embodiment, by reducing the levels of such free radicals as hydroxyl radicals, Olive-C60 is able to alleviate the damage of the cell membrane system of the organism, and protect the organism from secondary harm caused by such chemotherapeutic drugs as cyclophosphamide.

Effects of Olive-C60 on blood biochemical indexes in the second course on an intravital level: On the 18th day of the second course of the experiment, whole blood was taken from orbits, serums were obtained by centrifugation, and blood biochemical indexes were tested.

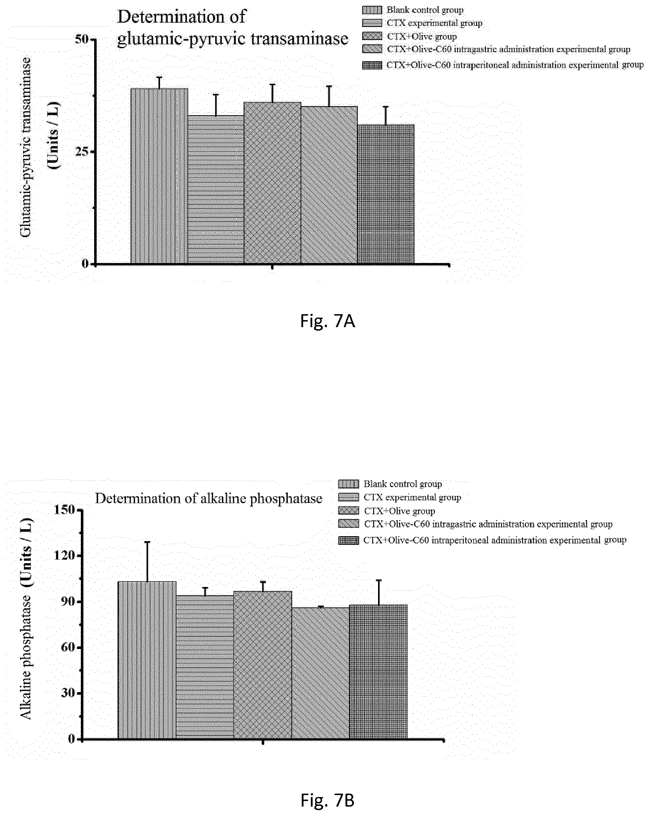

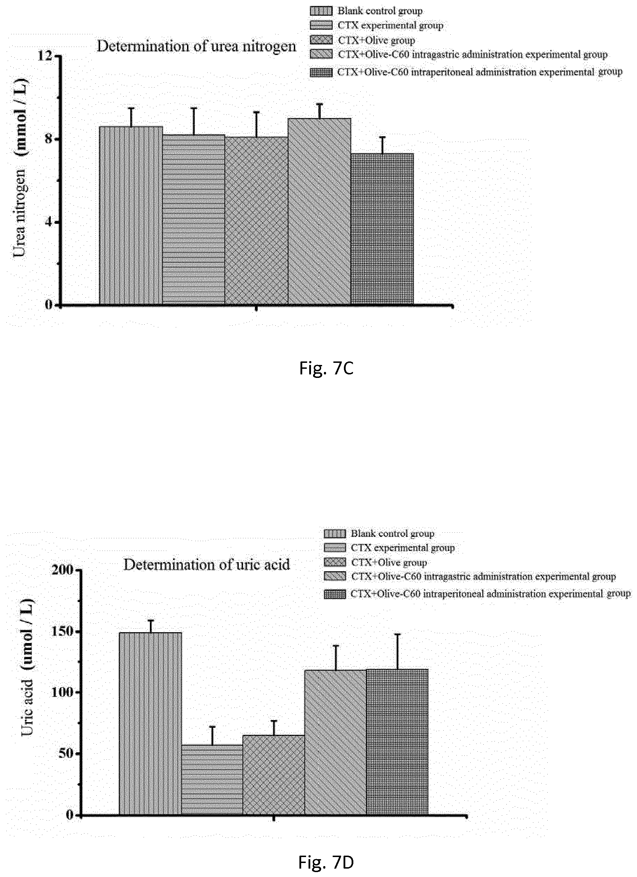

Corresponding detection results are as shown in Figures 7A-7D. As can be learned from Figures 7A-7D, after the multiple-course treatment process is completed, levels of glutamic-pyruvic transaminase, alkaline phosphatase and urea nitrogen of different experimental groups are almost the same and are all recovered to normal states. Attention should be paid to the level of uric acid. Specifically, the level of uric acid in the serum of mice in the cyclophosphamide experiment group is far lower than that in the serum of mice in the blank control group. Moreover, if the concentration of uric acid as a reducing agent is reduced, organism motion would produce a substantial amount of oxygen, thereby leading to a decline in its ability to scavenge free radicals, of which toxic effects might lead to a series of damage, including impairment of renal functions, hepatic injury, etc. However, the content of uric acid in the serum of mice injected intragastrically or intraperitoneally with Olive-C60 is almost comparable to that in the serum of mice in the blank control group, indicating that Olive-C60 has well protected the free radical-scavenging system of the organism, thereby protecting various visceral organs of the organism and functions thereof.

### Embodiment 4 Preparation of water-soluble hydroxylated gadolinium metallofullerene

1) 100 mg of Gd@C82 solid powder was added into a 100 ml single-necked flask, into which 7 ml of hydrogen peroxide aqueous solution having a volume fraction of 30% and 3 ml of sodium hydroxide aqueous solution having a concentration of 2 M were added respectively; the mixture was heated up to 70 °C and reacted for 2-5 hours in an oil bath;
2) after the reaction, small molecules were removed with a dialysis bag having a M.W. of 3,500, and monitoring was conducted using a conductivity meter until the dialysis was completed; thereafter, a resultant product was concentrated to obtain the water-soluble hydroxylated metallofullerene. As determined by DLS, the average grain size of the water-soluble hydroxylated metallofullerene in the aqueous solution is 140 nm, and its grain size distribution is uniform. The diagram of grain size distribution is as shown in Figure 8, and the water-soluble hydroxylated metallofullerene obtained in this embodiment is hereinafter referred to as GFNC.

### Embodiment 5 Metabolism distribution of GFNC in a living organism

1) Pre-test treatment:
   Animal models: BALB/c mice aged 4-5 weeks were selected, and 10⁶ mouse hepatocarcinoma cells (H22 cells) (available from the Cell Resource Center of Peking Union Medical College Hospital) of a volume of 100 µl were inoculated into their right thighs; the experiment was conducted 5-7 days after the inoculation when the sizes of tumors reached about 5 mm;
2) Test:
   150 µL of water-soluble hydroxylated gadolinium metallofullerene having a concentration of 1 mM was injected intravenously; thereafter, mice were sacrificed by cervical dislocation 15 minutes, 30 minutes, 45 minutes, 1 hour, 4 hours, 24 hours, 7 days, 15 days and 30 days after the injection, respectively, and dissection was conducted immediately for them to take their main visceral organs (hearts, livers, spleens, lungs, kidneys, intestines, stomachs, etc.), brains, tibias and muscles. Portions of such parts as the visceral organs, brains and muscles were taken and weighed; and tibias were weighed, thereafter, bone marrow in the tibias of mice was flushed out using PBS, and the tibias were weighed again to calculate the mass of the bone marrow. After the above tissues were digested, filtration was conducted using a 200 nm filtering membrane;
3) Testing conducted using an ICP-MS method for determining the concentration of gadolinium ions: a Gd³⁺ standard solution having a concentration of 100 ppb was taken and formulated into standard solution samples with their respective concentrations being 0 ppb, 30 ppb, 50 ppb, 100 ppb, 200 ppb and 300 ppb, wherein these standard solution samples were taken as standard samples for the ICP-MS test; then, the concentrations of Gd³⁺ in the above digestion solutions were measured respectively; thereafter, the concentrations of gadolinium ions in different tissues of equal mass were calculated by means of conversion formulas. 6 mice were assigned to each parallel experiment, and average values were taken.

Test results are as shown in Figure 9. As can be learned from Figure 9, the water-soluble hydroxylated gadolinium metallofullerene material is able to be significantly enriched in the bone within 1 hour, the concentration of which is only slightly lower than that in the liver; however, as time progresses, the concentration of gadolinium in the bone is able to be maintained at a high level, but 30 days later, the concentration of this material in the bone is obviously reduced, indicating that this material is metabolizable, which is consistent with the fact that fullerene is metabolizable on an intravital level.

The relative contents of the water-soluble hydroxylated gadolinium metallofullerene obtained in Embodiment 4 in the bone marrow and sclerotin of mice are as shown in Table 1. As can be learned from Table 1, sclerotin is separated from the bone marrow, and contents of the water-soluble hydroxylated gadolinium metallofullerene in the bone marrow and sclerotin of mice were measured respectively. 24 hours after the injection of samples, the content of gadolinium in the bone marrow is 5.84±0.58 ng/mg, which is much higher than that in the sclerotin (0.45±0.044 ng/mg). This further indicates that the material is featured by targeted enrichment in the bone marrow.

**Table 1 Relative Contents of Water-soluble Hydroxylated Gadolinium Metallofullerene in the Bone Marrow and Sclerotin of Mice**

| Different time points after injection | Contents of Gadoliniumin the bone marrow ( ng/mg ) | Contents of Gadoliniumin in the sclerotin (ng/mg) |
|---|---|---|
| 5 Minutes | 1.182±0.187 | 0.098±0.016 |
| 15 Minutes | 1.198±0.051 | 0.099 ±0.004 |
| 30 Minutes | 2.652±0.096 | 0.221±0.008 |
| 45 Minutes | 2.679±0.047 | 0.223±0.004 |
| 1 Hour | 3.521±0.298 | 0.293±0.025 |
| 4 Hours | 4.621±0.109 | 0.385±0.009 |
| 24 Hours | 5.388±0.528 | 0.449±0.044 |
| 7 Days | 8.185±0.388 | 0.515±0.032 |
| 15 Days | 5.538±0.512 | 0.461±0.043 |
| 30 Days | 4.431±0.514 | 0.369±0.043 |

### Embodiment 6 Metabolism distribution of GFNC in a living organism as determined using a radioactive labeling method

### 1) Pre-test treatment:

Animal models: BALB/c mice aged 4-5 weeks were selected and 10⁶ mouse hepatocarcinoma cells (H22 cells) were inoculated into their right thighs; the experiment was conducted 5-7 days after the inoculation when the sizes of tumors reached about 5 mm.

### 2) Iodine labeling:

Labeling conditions: substrate: ∼5 mg/ml, 1 ml; pH=8, H₃PO₄/NaOH; ¹³¹I: 5.0 MBq, 20 µL; Chloramine-T: 20 µL, 3 mg/mL; Reaction temperature: 30 °C; Reaction time: 3 hours; Terminating agent: Na₂S₂O₅; Labeling rate: ∼80%; Radiochemical purity: ≥99%.

### 3) Testing conducted using the ¹³¹I radiolabeling method:

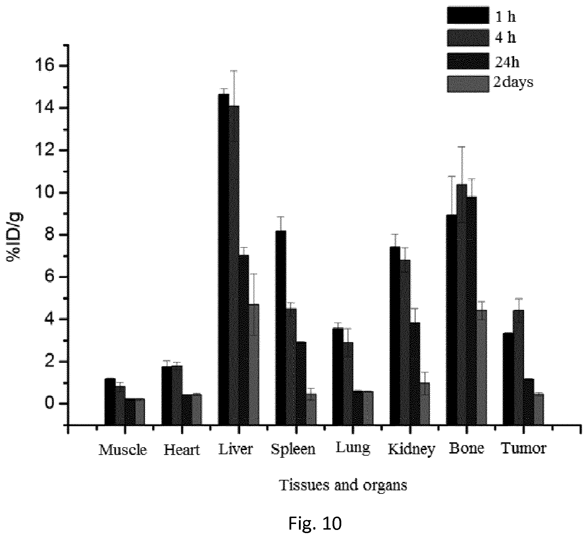

Hydroxyl groups present on the surface of the water-soluble hydroxylated gadolinium metallofullerene is able to carry out replacement reaction with ¹³¹I, and γ-rays emitted by ¹³¹I is able to be used to detect metabolic behaviors of these groups within an organism with a high sensitivity. Specifically, a chloramine-T aqueous solution (10 mg/mL) was first used to oxidize Na ¹³¹I solution of 37 MBq (the volume ratio was 2:5) for 10 minutes, a sample to be labeled was added, and oscillating reaction was conducted at room temperature for 2 hours. After the reaction was completed, a resultant reaction solution was subjected to separation and purification using Sephadex G-25 gel column so as to remove inorganic salt ions, thereby obtaining radioiodine-131 labeled samples (radiochemical purity≥97%). 50 µL of activated solution was injected respectively into mice with tumors via caudal veins; then, mice were sacrificed by cervical dislocation 1.0 hour, 4.0 hours, 24 hours and 48 hours after the injection, respectively; thereafter, dissection was conducted to take out their main tissues and organs (hearts, livers, spleens, lungs, kidneys, tumors, brains, bones, fleshes, etc.) for gamma counting; after that, the contents of labeled samples in various organs were counted. Test results are as shown in Figure 10. As can be learned from Figure 10, the obtained results are similar to those obtained using the ICP ion concentration testing method, which corroborates that the water-soluble hydroxylated gadolinium metallofullerene material is able to be significantly enriched in bones. Also, the bone marrow of mice is flushed out of tibias, and measurements are conducted respectively for the masses of the bone marrow and sclerotin as well as the intensity of γ counting. As can be seen from Table 2, the water-soluble hydroxylated gadolinium metallofullerene is mainly enriched in the bone marrow.

**Table 2 Relative Contents of Water-soluble Hydroxylated Gadolinium Metallofullerene Obtained in Embodiment 4 present in the Bone Marrow and Sclerotin of Mice**

| Differernt time points after injection | Radioactivity of ¹³¹I in the bone marrow | Radioactivity of ¹³¹I in the sclerotin |
|---|---|---|
| 1 Hour | 8.25±1.29 | 0.75±0.07 |
| 4 Hours | 9.63±1.12 | 0.88±0.10 |
| 24 Hours | 9.12±0.92 | 0.83±0.04 |
| 48Hours | 4.4±0.688 | 0.41±0.08 |

### Embodiment 7 Determination of the enrichment amount of GFNC in the bone marrow by means of electronic probes

### 1) Pre-test treatment:

Animal models: BALB/c mice aged 4-5 weeks were selected, and 10⁶ mouse hepatocarcinoma cells (H22 cells) were inoculated into their right thighs; the experiment was conducted 5-7 days after the inoculation when the sizes of tumors reached about 5 mm.

Experimental group: BALB/c mice aged 4-5 weeks were selected, and 10⁶ mouse hepatocarcinoma cells (H22 cells) were inoculated into their right thighs; 150 µL of water-soluble hydroxylated metallofullerene having a concentration of 900 ppb was injected intravenously 5-7 days after the inoculation when the sizes of tumors reached about 5 mm.

### 2) Test:

Mice experiment: 150 µL of water-soluble hydroxylated gadolinium metallofullerene having a concentration of 1 mM was injected intravenously; before the injection, and 0.5 hour, 1 hour, 4 hours and 24 hours after the injection, mice were sacrificed respectively by cervical dislocation, and dissection was conducted immediately for them to take bilateral tibias of mice. Thereafter, bone marrow in the tibias of mice was flushed out immediately using PBS, and was evenly applied on the surface of an electrically conducting paste; thereafter, the content of gadolinium elements was detected using an electronic probe instrument.

Water-soluble hydroxylated gadolinium metallofullerene sample experiment: a water-soluble hydroxylated gadolinium metallofullerene sample of aqueous solution was evenly applied on the surface of the electrically conducting paste, and after it was dried, testing was conducted using the electronic probe instrument.

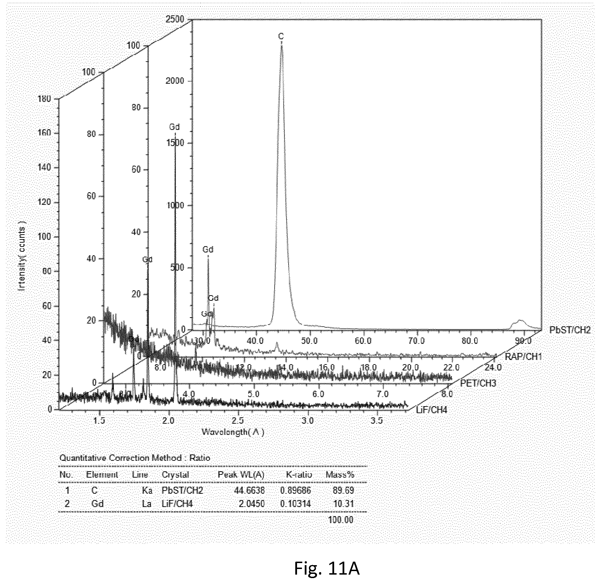

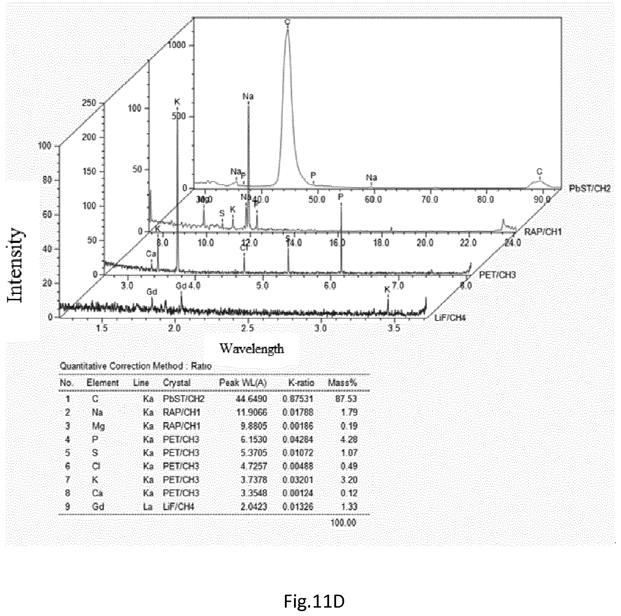

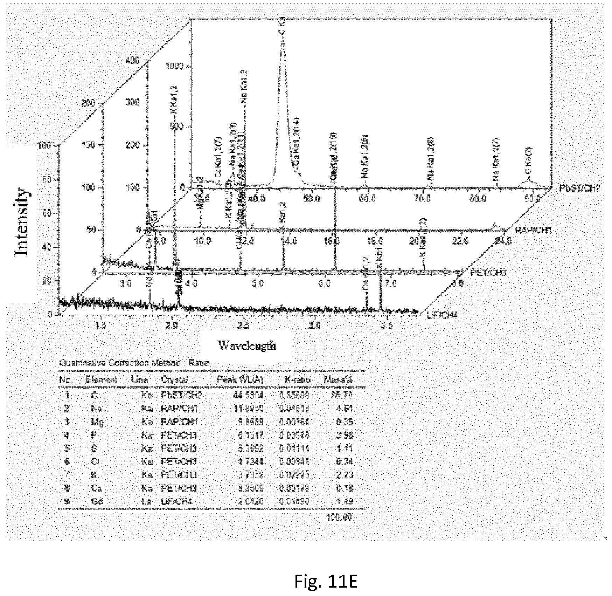

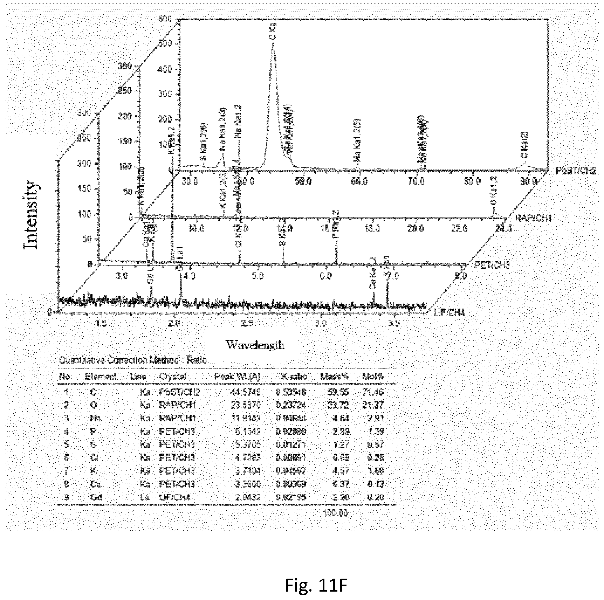

Corresponding test results are as shown in Figures 11A-F. As can be seen from Figure 11A, the position of 2.04A represents the position where gadolinium ions of the water-soluble hydroxylated gadolinium metallofullerene are located in the electronic probe experiment, which provides a reference for the content of gadolinium ions in the bone marrow of mice.

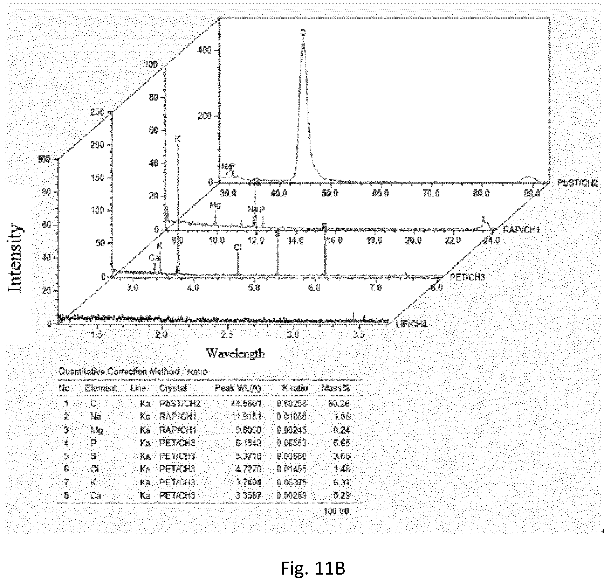

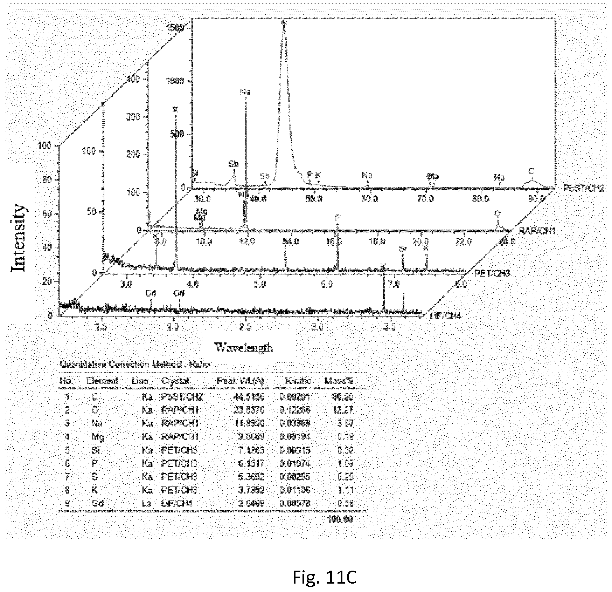

As can be seen from Figure 11B, before the water-soluble hydroxylated gadolinium metallofullerene injection, gadolinium ions can hardly be detected in the bone marrow of mice. As can be seen from Figures 11C-11F, after the water-soluble hydroxylated gadolinium metallofullerene injection, the content of gadolinium detected in the bone marrow of mice increases as time progresses. Specifically, this content is 0.58% (measured in terms of mass percent, and similarly hereinafter) at 0.5 hour, 1.33% at 1 hour, 1.49% at 4 hours, and 2.2% at 24 hours. This electronic probe experiment also further corroborates that the material herein can quickly enter the bone marrow of mice and be enriched therein.

### Embodiment 8 Study on the toxicity of GFNC on an intravital level

Water-soluble hydroxylated gadolinium metallofullerene group: 150 µL of water-soluble hydroxylated gadolinium metallofullerene having a concentration of 1 mM was injected intravenously; then, 15 minutes, 30 minutes, 45 minutes, 1 hour, 4 hours, 24 hours, 7 days, 15 days and 30 days after the injection, mice were fetched and their eye balls were removed, and blood was taken respectively from their orbits; thereafter, the blood was placed in a 5 ml centrifuge tube and a centrifugation was carried out for 15 minutes at a rotational speed of 3,500 rpm; after that, a resultant serum was transferred into a 200 µL centrifuge tube, and blood biochemical indexes (e.g., ALT, ALP, AST, BUN, LDH, etc.) of the mice were detected in a blood biochemical instrument. Changes in the weight of mice were observed for a period of 30 days.

Normal saline group: This group was processed in the same way as the metallofullerene group for comparison, and the water-soluble hydroxylated gadolinium metallofullerene was replaced with normal saline.

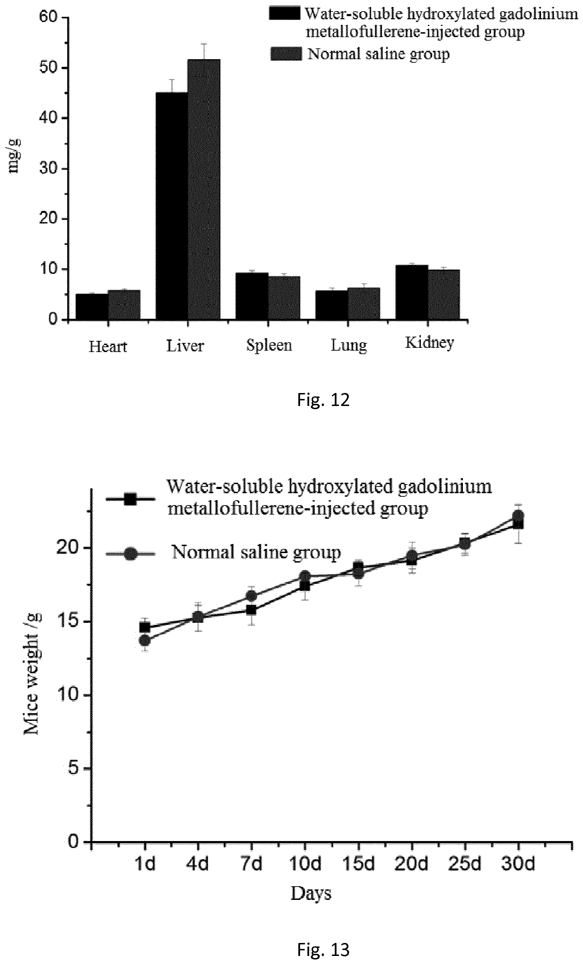

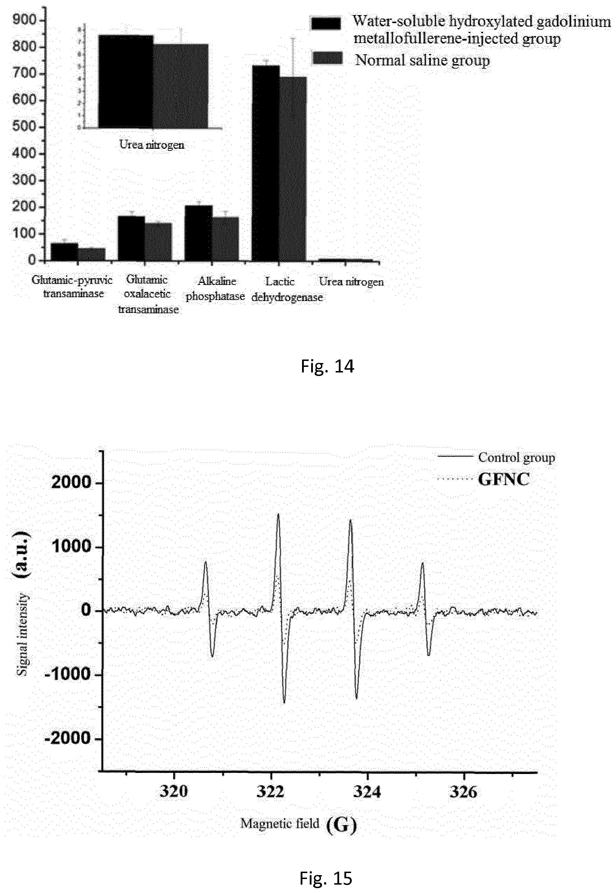

Corresponding test results are as shown in Figures 12, 13 and 14. There is no obvious difference between the metallofullerene group and the normal saline group with respect to either the weight of mice or short-term and long-term coefficients for visceral organs, indicating that this material has low toxicity, and can be further used for clinical studies. As can be learned from Figure 14, it is obvious that as compared with the normal saline group, the metallofullerene group does not exhibit obvious degradation in hepatic and renal functions, indicating that it has low toxicity. This corroborates that the material herein is safe and non-toxic.

### Embodiment 9 In-vitro scavenging effects of GFNC on free radicals

### 1) Experiment for detecting free radical intensity using electron spin-resonance (ESR):

A method in which hydroxyl radicals were generated by means of UV induction was employed. Control group: 50 µL of hydrogen peroxide having a mass concentration of 37%, 50 µL of PBS buffer solution (pH=7.4) and a trace amount (0.133 mM) of dimethyl pyridine N-oxide (DMPO, free radical trapping agent) solution were mixed together, and a resultant mixture was irradiated for 8 minutes using 280 nm UV light; a signal indicative of hydroxyl radicals is able to be generated; experimental group: 50 µL of hydrogen peroxide having a mass concentration of 37%, 50 µL of PBS buffer solution (pH=7.4) and a trace amount (0.133 mM) of dimethyl pyridine N-oxide (DMPO, free radical trapping agent) solution were mixed together, and 10 µL of water-soluble hydroxylated gadolinium metallofullerene aqueous solution having a concentration of 20 µM was added immediately; thereafter, irradiation was conducted for 8 minutes using 280 nm UV light, and the signal intensity of free radicals was detected.

Corresponding detection results are as shown in Figure 15. As can be learned from Figure 15, in view of the experimental group, GFNC is capable of effectively quenching free radicals, which are generated when hydrogen peroxide is irradiated by UV light, at a concentration of only 20 µM, thereby playing a role in protecting cells from being damaged by free radicals generated by hydrogen peroxide.

### Embodiment 10 Effects of GFNC on the protection of cells from being killed by free radicals generated by hydrogen peroxide on a cellular level

Bone marrow cells (FDC-P1) of mice were selected as the research object, and the culture medium thereof was high glucose DMEM added with cytokine IL-3. Bone marrow cells of mice were inoculated into 8×6 wells of a 96-well plate at a concentration of ca.1x10⁴/well; wherein, a negative control group was assigned with 6 wells; a positive control group was also assigned with 6 wells; an experimental group used 7 different concentrations of GFNC in total, and 6 wells were assigned for GFNC at each concentration. The experiment was repeated for three times. The negative control group was a culture medium inoculated merely with bone marrow cells of mice, but without hydrogen peroxide and GFNC; the positive control group was added merely with 10 µL of GFNC having a concentration of 30 µM, but without hydrogen peroxide solution; The experimental group was added, in parallel, with 20 µL of hydrogen peroxide solution having a concentration of 100 µM, 90 µL of the culture medium, and different concentrations of GFNC (such that GFNC final concentrations were, respectively, 0.5 µM, 1 µM, 2.5 µM, 5 µM, 10 µM, 20 µM and 30 µM).

The adding sequence of the negative control group was as follows: neither hydrogen peroxide nor GFNC was added; rather, they were replaced with PBS of the same volume.

The adding sequence of the positive control group was as follows: hydrogen peroxide was replaced with PBS of the same volume, and bone marrow cells of mice were incubated for 1 hour; thereafter, GFNC was added, and incubation was conducted for 3 hours.

The adding sequence of the experimental group was as follows: hydrogen peroxide and the culture medium were added first, a resultant mixture and bone marrow cells of mice were incubated for 1 hour; thereafter, the hydrogen peroxide solution was sucked out, and washing was conducted for three times with PBS buffer solution so as to wash off the remaining hydrogen peroxide solution; then, GFNC of a certain concentration and the cell culture medium were added; after bone marrow cells of mice were incubated for 3 hours, the culture medium containing GFNC was sucked out, and washing was conducted for three times with PBS buffer solution; finally, the cell culture medium was added, and incubation was continued for 24 hours in a cell incubator; then, cell activities were detected using CCK-8.

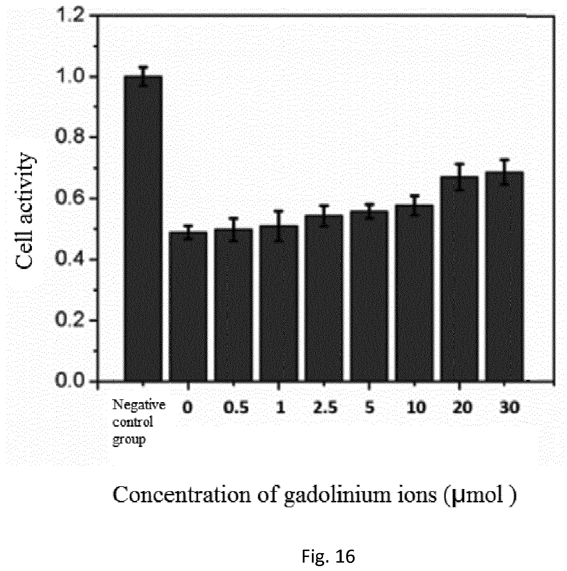

Corresponding detection results are as shown in Figure 16. As can be learned from Figure 16, when 20 µL of hydrogen peroxide solution having a concentration of 100 µM is added, hydrogen peroxide exerts certain killing effects on bone marrow cells of mice, while cell activities increase as the addition amount of water-soluble hydroxylated gadolinium metallofullerene is increased gradually. This indicates that water-soluble hydroxylated gadolinium metallofullerene is effective for scavenging free radicals, and is able to protect cells from its influence on a cellular level. Meanwhile, as for the positive control group added merely with water-soluble hydroxylated gadolinium metallofullerene, but without hydrogen peroxide, its cell activities are slightly higher than those of the negative control group, which indicates that the material is not cytotoxic.

### Embodiment 11 Protection effects of GFNC on myelosuppression on an intravital level

Animal model: ICR mice aged 4-5 weeks were selected and assigned randomly into 4 groups with 6 mice per group. These groups were, respectively, a blank control group, a GFNC experimental group, an X-ray irradiation experimental group and an X-ray+GFNC experimental group. 10⁶ mouse hepatocarcinoma cells (H22 cells) were inoculated into right thighs of mice, and the experiment was conducted 5-7 days after the inoculation when the sizes of tumors reached about 5 mm.

Blank control group: The injected drugs in the experimental groups were replaced with equal volumes of normal saline, and equal volumes of normal saline were injected intravenously.

GFNC experimental group: Mice were injected with a GFNC aqueous solution (1 mM) via caudal veins at a dosage of 0.004 mmol of GFNC/kg of body weight of mice.

X-ray irradiation experimental group: Mice were treated with 6Gy X-ray whole body radiation in one time.

X-ray+GFNC experimental group: Mice were treated with 6Gy X-ray whole body radiation in one time, and GFNC solution was injected intravenously at a dosage of 0.004 mmol of GFNC/kg of body weight of mice.

Injection of drugs was initiated on the 7th day after tumor inoculation, which was marked as the first day of the experiment. Intravenous injection of GFNC was conducted once a day for 5 consecutive days, and X-ray irradiation was conducted on the first day; thereafter, blood (20 µl) was taken respectively from orbits of mice on the 4th day, the 7th day, the 10th day, the 14th day and the 17th day, and was placed in a 3 ml centrifuge tube, and blood routine examination was conducted using a blood cell automatic analyzer, wherein the main indexes related to myelosuppression are white blood cell counts (WBC), platelet counts (PLT), hemoglobin determination (HGB) and monocyte proportion (MO%).

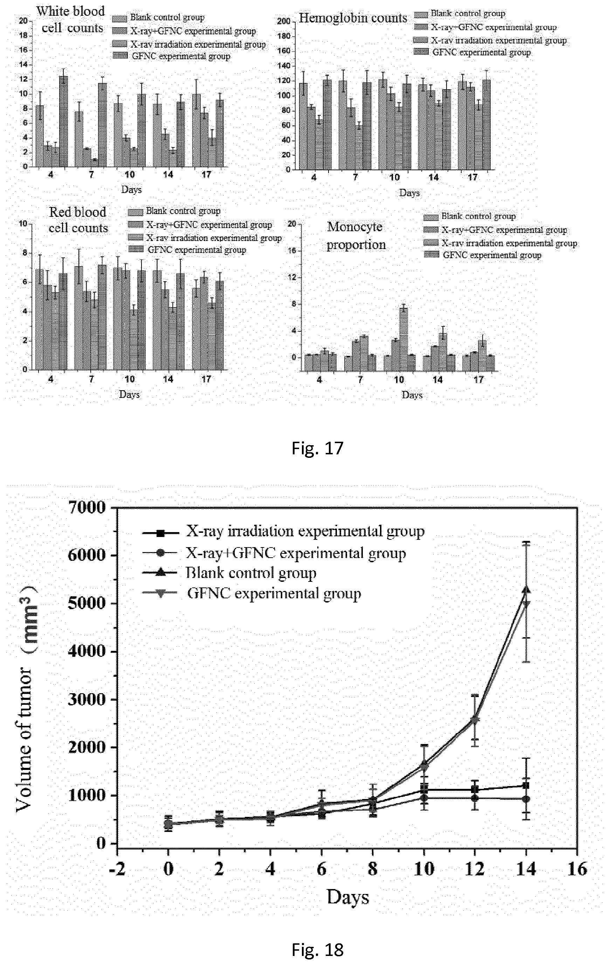

Corresponding detection results are as shown in Figure 17. It can be learned from Figure17 that as compared with the blank control group, the X-ray irradiation experimental group exhibits the following effects: the myelosuppression-related indexes of mice, namely white blood cells, platelets and hemoglobin, are subjected to varying degrees of reduction, wherein the reduction in white blood cells is the most remarkable. The phenomenon of abnormal hyperplasia is observed in their monocytes, which indicates that bone marrow is damaged, moreover, significant abnormalities are also observed in related indexes; however, as for the mice in the X-ray+GFNC experimental group, the numbers of their white blood cells, platelets and hemoglobin are greatly increased as compared with those of the X-ray experimental group owing to protective effects of GFNC, and the number of monocytes is closer to that of the blank group group, moreover, as time progresses, related indexes are getting closer to those of normal mice, indicating that GFNC has obviously protective effects on the myelosuppression of mice caused by radiotherapeutic X-rays.

### Embodiment 12 Influences of GFNC on the therapeutic effects of X-ray radiotherapy on tumors on an intravital level

Animal model: ICR mice aged 4-5 weeks were selected and assigned randomly into 4 groups with 6 mice per group. These groups were, respectively, a blank control group, a GFNC experimental group, an X-ray irradiation experimental group and an X-ray+GFNC experimental group. 10⁶ mouse hepatocarcinoma cells (H22 cells) were inoculated into right thighs of mice, and the experiment was conducted 5-7 days after the inoculation when the sizes of tumors reached about 5 mm.

Blank control group: The injected drugs in the experimental groups were replaced with equal volumes of normal saline.

X-ray irradiation experimental group: Mice were treated with X-ray whole body radiation for one time at an irradiation dosage of 6Gy.

Metallofullerene derivative (GFNC) experimental group: Mice were injected intravenously with GFNC solution at a dosage of 0.04 mmol of Gd³⁺/kg of body weight of mice.

X-ray+GFNC experimental group: Mice were treated with X-ray whole body radiation for one time at an irradiation dosage of 6Gy, and GFNC solution was injected intravenously at a dosage of 0.04 mmol of Gd³⁺/kg of body weight of mice.

Injection of drugs was initiated on the 7th day after tumor inoculation, which was marked as the first day of the experiment. Intravenous injection of GFNC was conducted once a day for 5 consecutive days, and weights of mice and tumor diameters were measured every two days.

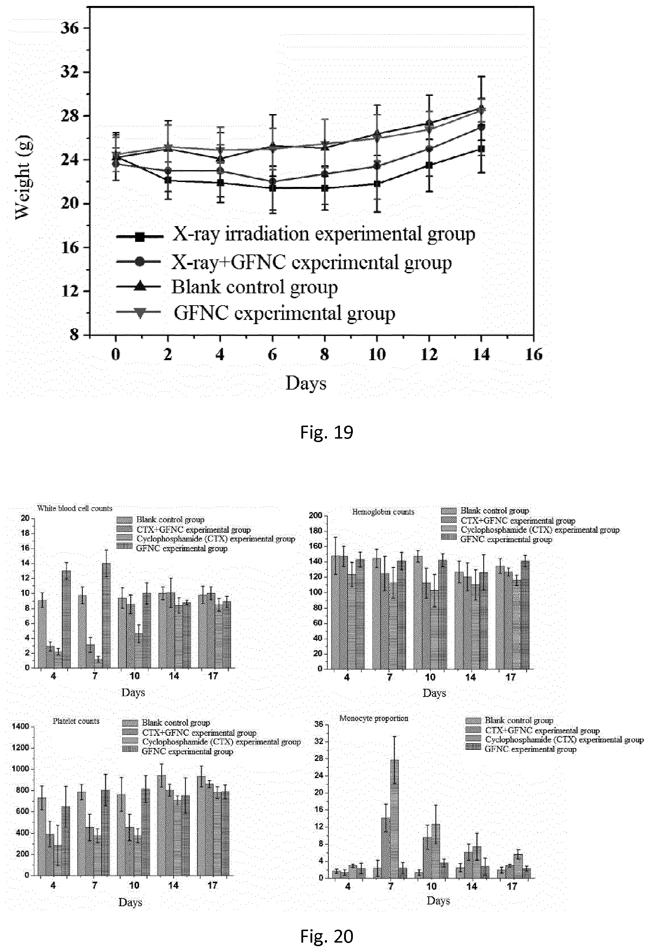

Corresponding detection results are as shown in Figures 18 and 19. It can be learned from Figure 18 that as compared with the X-ray experimental group, the tumor diameters of the mice in the X-ray+GFNC experimental group turn out to be smaller, but not bigger. This indicates that GFNC is able to significantly inhibit the myelosuppression toxicity caused by radiotherapy without affecting the therapeutic effects of radiotherapy on mouse tumors, i.e. that there is synergy between GFNC and radiotherapy. Referring to Figure 19, as can be found through comparison of weight changes of mice in different groups, the weight of mice in the X-ray experimental group drops obviously in the initial stage of drug injection; thereafter, as drug injection is terminated, mice put on some weight owing to their own restoration functions, but this group is still the lightest in weight; however, as for mice in the X-ray+GFNC experimental group, their weights drop slightly in the initial stage of drug injection, which, however, bounce back obviously in the later stage, and approach to those of normal mice. This indicates that GFNC is able to protect mice from side effects caused by radiotherapeutic rays to the greatest extent. It can protect mice from myelosuppression toxicity while exhibiting low toxicity, which can be further used for clinical studies.

### Embodiment 13 Protection effects of water-soluble hydroxylated gadolinium metallofullerene on myelosuppression on an intravital level

Animal model: ICR mice aged 4-5 weeks were selected and assigned randomly into 4 groups with 6 mice per group. These groups were, respectively, a blank control group, a GFNC experimental group, a cyclophosphamide (CTX) experimental group and a CTX+GFNC experimental group. 10⁶ mouse hepatocarcinoma cells (H22 cells) were inoculated into right thighs of mice, and the experiment was conducted 5-7 days after the inoculation when the sizes of tumors reached about 5 mm.

Blank control group: The drugs injected either intragastrically or intraperitoneally in the experimental groups were replaced with equal volumes of normal saline.

GFNC experimental group: Mice were injected with a GFNC aqueous solution (1 mM) via caudal veins at a dosage of 0.004 mmol of GFNC/kg of body weight of mice.

Cyclophosphamide (CTX) experimental group: Mice were injected intraperitoneally with a CTX solution at a dosage of 60 mg/kg of body weight of mice.

CTX+GFNC experimental group: Mice were injected intraperitoneally with a CTX solution at a dosage of 60 mg/kg of body weight of mice, and GFNC solution was injected intravenously at a dosage of 0.004 mmol of GFNC/kg of body weight of mice.

Injection of drugs was initiated on the 7th day after tumor inoculation, which was marked as the first day of the experiment. Intraperitoneal injection of CTX or intravenous injection of GFNC was conducted once a day for 5 consecutive days; thereafter, blood (20 µl) was taken respectively from orbits of mice on the 4th day, the 7th day, the 10th day, the 14th day and the 17th day, and was placed in a 3 ml centrifuge tube, and the blood routine examination was conducted using a blood cell automatic analyzer, wherein the main indexes related to myelosuppression are white blood cell counts (WBC), platelet counts (PLT), hemoglobin determination (HGB) and monocyte proportion (MO%).

Corresponding detection results are as shown in Figure 20. It can be learned from Figure 20 that as compared with the blank control group, the cyclophosphamide (CTX) experimental group exhibits the following effects: the myelosuppression-related indexes of mice, namely white blood cells, platelets and hemoglobin, are subjected to varying degrees of reduction, wherein the reduction in white blood cells is the most remarkable. The phenomenon of abnormal hyperplasia is observed in their monocytes, which indicates that their bone marrow is damaged, moreover, significant abnormalities are also observed in related indexes; however, as for the mice in the CTX+GFNC experimental group, the numbers of their white blood cells, platelets and hemoglobin are greatly increased as compared with those of the cyclophosphamide (CTX) experimental group owing to protective effects of GFNC, and the number of monocytes is closer to that of the blank control group. Moreover, as time progresses, related indexes are getting closer to those of normal mice. This indicates that GFNC has obviously protective effects on the myelosuppression of mice caused by chemotherapeutic drug CTX.

### Embodiment 14 Influence of water-soluble hydroxylated gadolinium metallofullerene on the therapeutic effects of chemotherapeutic drug CTX on tumors on an intravital level and toxic experiment

Animal model: ICR mice aged 4-5 weeks were selected and assigned randomly into 4 groups with 6 mice per group. These groups were, respectively, a blank control group, a GFNC experimental group, a cyclophosphamide (CTX) experimental group and a CTX+GFNC experimental group. 10⁶ mouse hepatocarcinoma cells (H22 cells) were inoculated into right thighs of mice, and the experiment was conducted 5-7 days after the inoculation when the sizes of tumors reached about 5 mm.

Blank control group: The injected drugs in the experimental groups were replaced with equal volumes of normal saline.

Cyclophosphamide (CTX) experimental group: Mice were injected intraperitoneally with a CTX solution at a dosage of 60 mg/kg of body weight of mice.

Metallofullerene derivative (GFNC) experimental group: Mice were injected intravenously with a GFNC solution at a dosage of 0.04 mmol of Gd³⁺/kg of body weight of mice.

CTX+GFNC experimental group: Mice were injected intraperitoneally with a CTX solution at a dosage of 60 mg/kg of body weight of mice, and GFNC solution was injected intravenously at a dosage of 0.04 mmol of Gd³⁺/kg of body weight of mice.

Injection of drugs was initiated on the 7th day after tumor inoculation, which was marked as the first day of the experiment. Intraperitoneal injection of CTX or intravenous injection of GFNC was conducted once a day for 5 consecutive days, and weights of mice and tumor diameters were measured every two days; thereafter, on the 17th day, mice were fetched and their eye balls were removed, and blood was taken respectively from their orbits; then, the blood was placed in a 5 ml centrifuge tube where it was centrifuged for 15 minutes at a rotational speed of 3,500 rpm; after that, a resultant serum was transferred into a 200 µL centrifuge tube, and blood biochemical indexes (e.g., ALT, ALP, AST, BUN, LDH and UA) of the mice were detected in a blood biochemical instrument; after mice were sacrificed by cervical dislocation, their main tissues and organs (hearts, livers, spleens, lungs and kidneys) were taken and weighed, and were then soaked in a 4% formalin solution; thereafter, histopathological section was conducted, and then, H&E staining was conducted. The blank control group, the cyclophosphamide (CTX) experimental group and the metallofullerene experimental group (GFNC) were processed in the same way for comparison.

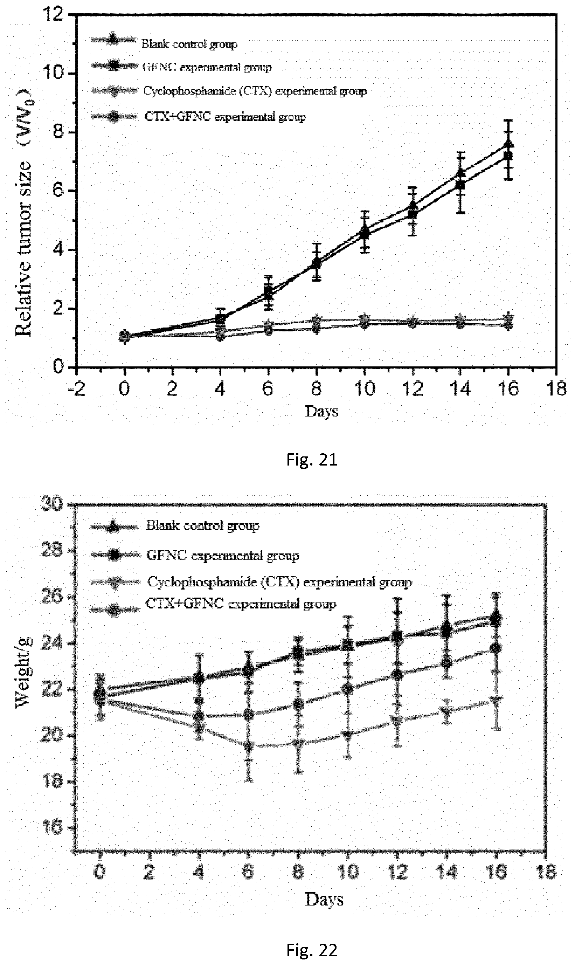

Corresponding detection results are as shown in Figures 21 and 22. It can be learned from Figure 21 that as compared with the cyclophosphamide (CTX) experimental group, the tumor diameters of the mice in the CTX+GFNC experimental group turn out to be smaller, but not bigger. This indicates that GFNC is able to significantly inhibit the myelosuppression toxicity caused by chemotherapeutic drug CTX without affecting the therapeutic effects of chemotherapeutic drug CTX on mouse tumors, i.e. that there is synergy between CTX and GFNC. Referring to Figure 22, as can be found through comparison of weight changes of mice in different groups, the weight of mice in the cyclophosphamide (CTX) experimental group drops obviously in the initial stage of drug injection; thereafter, as drug injection is terminated, mice put on some weight owing to their own restoration functions, but this group is still the lightest in weight; however, as for mice in the CTX+GFNC experimental group, their weights drop slightly in the initial stage of drug CTX injection, which, however, bounce back obviously in the later stage, and approach to those of normal mice. This indicates that GFNC is able to protect mice from side effects caused by the chemotherapeutic drug to the greatest extent, andit can protect mice from myelosuppression toxicity while exhibiting low toxicity, which can be further used for clinical studies.

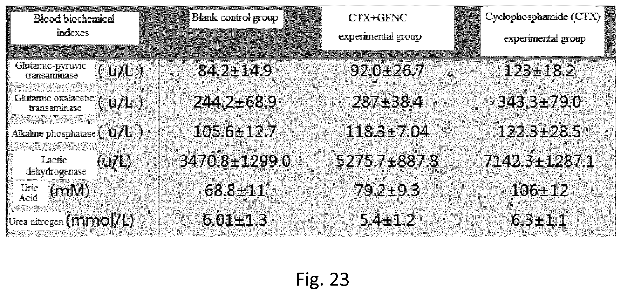

As can be learned from Figure 23, the enzyme activities of the CTX+GFNC experimental group derived from tests drop to a certain extent or level off as compared to the CTX experimental group. This indicates that GFNC has certain scavenging effects on reactive oxygen species in mice while exhibiting low toxicity.

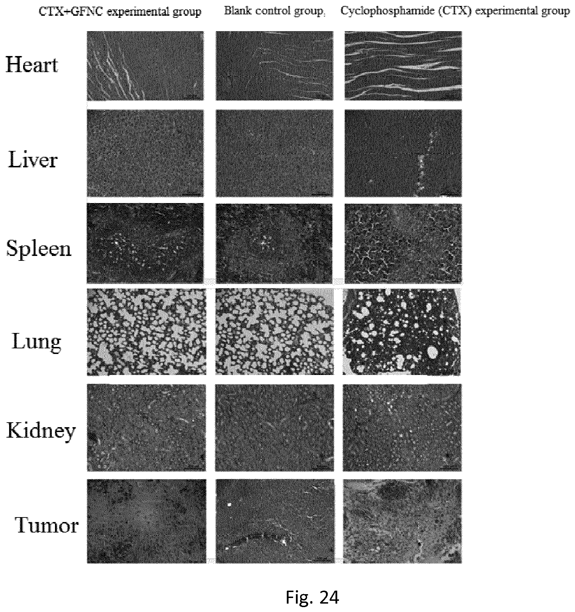

Referring to Figure 24, as can be learned from the comparison of pictures of histopathological sections, such phenomena as swelling, necrosis and inflammation are not observed in the main tissues and organs in the CTX+GFNC experimental group, and there is almost no difference between this group and the blank control group; as for mice in the cyclophosphamide (CTX) experimental group, their tissues and organs are damaged to a certain extent, moreover, as can be seen from pathological sections of tumor sites, tumor tissues of the blank control group are thriving, and tumor cells are dense; however, cells of tumor tissues in the CTX+GFNC experimental group and the CTX experimental group die in great quantity, and tissues are loose. This indicates that GFNC is safe and non-toxic, and exerts no influence on the therapeutic effects of chemotherapeutic drug CTX on tumors.

### Embodiment 15 Preparation of water-soluble hydroxylated hollow fullerene

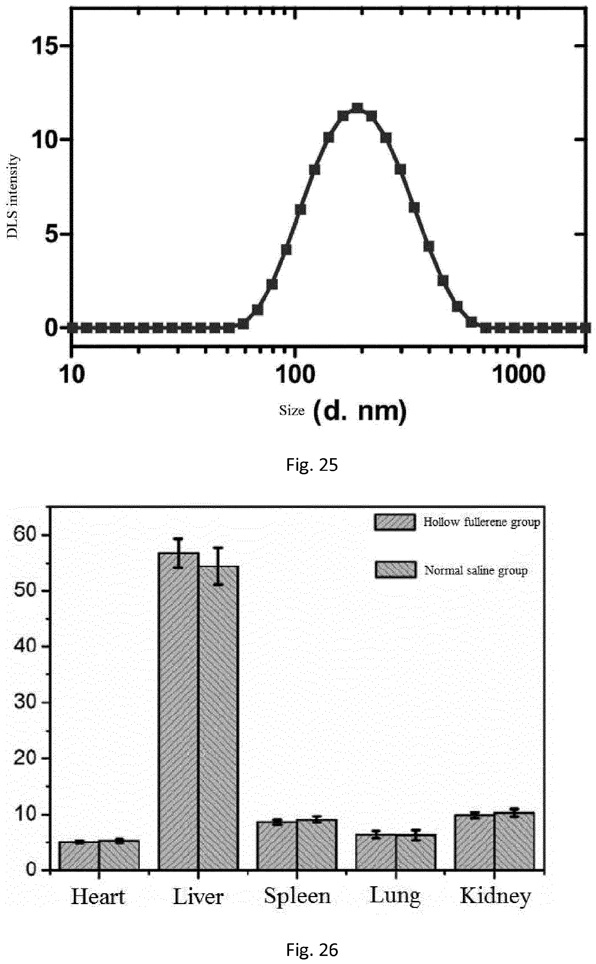

1) 100 mg of C60 solid powder was added into a 100 ml single-necked flask, into which 7 ml of hydrogen peroxide aqueous solution having a volume fraction of 30% and 3 ml of sodium hydroxide aqueous solution having a concentration of 2 M were added respectively; the mixture was heated up to 70 °C and reacted for 2-5 hours in an oil bath;
2) after the reaction, small molecules were removed with a dialysis bag having a M.W. of 3,500, and monitoring was conducted using a conductivity meter until the dialysis was completed; thereafter, a resultant product was concentrated to obtain the water-soluble hydroxylated hollow fullerene. As determined by DLS, the average grain size of the water-soluble hydroxylated hollow fullerene in the aqueous solution is 200 nm, and its grain size distribution is uniform. The diagram of grain size distribution is as shown in Figure 25.

### Embodiment 16 Research on the toxicity of water-soluble hydroxylated hollow fullerene on an intravital level

Water-soluble hydroxylated hollow fullerene group: 150 µL of water-soluble hydroxylated hollow fullerene having a concentration of 1 mM was injected intravenously; then, 30 days after the injection, mice were fetched and their eye balls were removed, and blood was taken from their orbits; thereafter, the blood was placed in a 5 ml centrifuge tube where it was centrifuged for 15 minutes at a rotational speed of 3,500 rpm; after that, a resultant serum was transferred into a 200 µL centrifuge tube, and blood biochemical indexes (e.g., ALT, ALP, AST, BUN, LDH, etc.) of the mice were detected in a blood biochemical instrument. Changes in the weight of mice were observed for a period of 30 days. Normal saline group: The group was processed in the same way as the hollow fullerene group for comparison, except for the water-soluble hydroxylated hollow fullerene was replaced with normal saline.

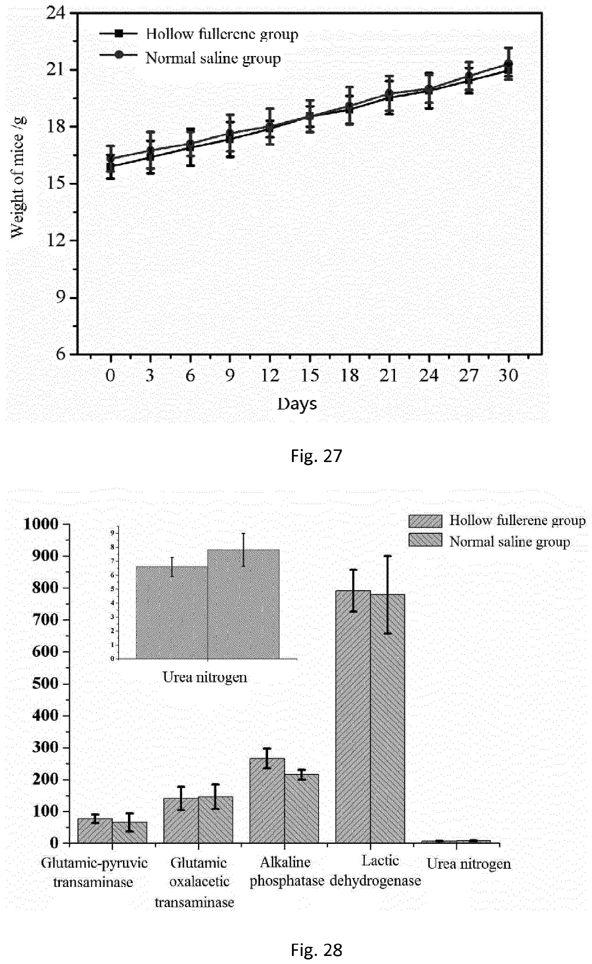

Corresponding test results are as shown in Figures 26, 27 and 28. As can be learned from Figures 26 and 27, there is no obvious difference between the hollow fullerene group and the normal saline group with respect to either the weight of mice or short-term and long-term coefficients for visceral organs. This indicates that this material has low toxicity, and thus can be further used for clinical studies. As can be learned from Figure 28, it is obvious that as compared with the normal saline group, the hollow fullerene group does not exhibit obvious degradation in hepatic and renal functions, indicating that it has low toxicity. This corroborates that the material herein is safe and non-toxic.

### Embodiment 17 In-vitro scavenging effects of water-soluble hydroxylated hollow fullerene on free radicals

### 1) Experiment for detecting free radical intensity using electron spin-resonance (ESR):

A method in which hydroxyl radicals were generated by means of UV induction was employed. Control group: 50 µL of hydrogen peroxide having a mass concentration of 37%, 50 µL of PBS buffer solution (pH=7.4) and a trace amount (0.133 mM) of dimethyl pyridine N-oxide (DMPO, free radical trapping agent) solution were mixed together, and a resultant mixture was irradiated for 8 minutes using 280 nm UV light; a signal indicative of hydroxyl radicals is able to be generated; experimental group: 50 µL of hydrogen peroxide having a mass concentration of 37%, 50 µL of PBS buffer solution (pH=7.4) and a trace amount (0.133 mM) of dimethyl pyridine N-oxide (DMPO, free radical trapping agent) solution were mixed together, and 10 µL of water-soluble hydroxylated hollow fullerene aqueous solution having a concentration of 20 µM was added immediately; thereafter, irradiation was conducted for 8 minutes using 280 nm UV light, and the signal intensity of free radicals was detected.

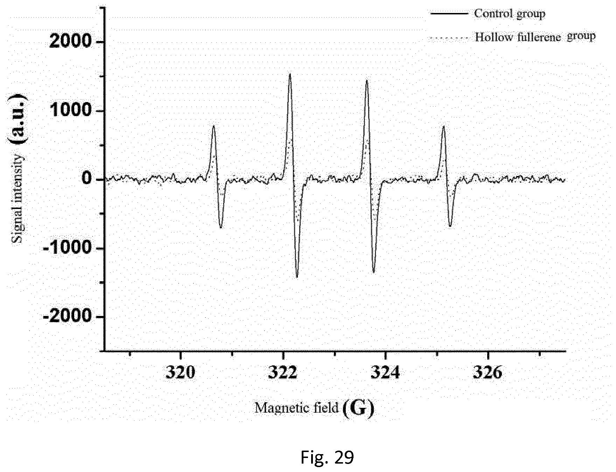

Corresponding detection results are as shown in Figure 29. As can be learned from Figure 29, in view of the experimental group, the hydroxylated hollow fullerene aqueous solution is capable of effectively quenching free radicals, which are generated when hydrogen peroxide is irradiated by UV light, at a concentration of only 20 µM, thereby playing a role in protecting cells from being damaged by free radicals generated by hydrogen peroxide.

### Embodiment 18 Effects of water-soluble hydroxylated hollow fullerene on the protection of cells from being killed by free radicals generated by hydrogen peroxide on a cellular level

Bone marrow cells (FDC-P1) of mice were selected as the research object, and the culture medium thereof was high glucose DMEM added with cytokine IL-3. Bone marrow cells of mice were inoculated into 8×6 wells of a 96-well plate at a concentration of ca.1x10⁴/well; wherein, a negative control group was assigned with 6 wells; a positive control group was also assigned with 6 wells; an experimental group used 7 different concentrations of hydroxylated hollow fullerene aqueous solutions in total, and 6 wells were assigned for each concentration of hydroxylated hollow fullerene aqueous solution. The experiment was repeated for three times. The negative control group was a culture medium inoculated merely with bone marrow cells of mice, but without hydrogen peroxide and hydroxylated hollow fullerene aqueous solution; the positive control group was added merely with 10 µL of hydroxylated hollow fullerene aqueous solution having a concentration of 30 µM, but without hydrogen peroxide solution; the experimental group was added, in parallel, with 20 µL of hydrogen peroxide solution having a concentration of 100 µM, 90 µL of the culture medium, and different concentrations of hydroxylated hollow fullerene aqueous solutions (such that final concentrations of hydroxylated hollow fullerene aqueous solutions were, respectively, 0.5 µM, 1 µM, 2.5 µM, 5 µM, 10 µM, 20 µM and 30 µM).

The adding sequence of the negative control group was as follows: neither hydrogen peroxide nor hydroxylated hollow fullerene aqueous solution was added; rather, they were replaced with PBS of the same volume. The adding sequence of the positive control group was as follows: hydrogen peroxide was replaced with PBS of the same volume, and bone marrow cells of mice were incubated for 1 hour; thereafter, the hydroxylated hollow fullerene aqueous solution was added, and incubation was conducted for 3 hours.

The adding sequence of the experimental group was as follows: hydrogen peroxide and the culture medium were added first, and bone marrow cells of mice were incubated for 1 hour; thereafter, the hydrogen peroxide solution was sucked out, and washing was conducted for three times with PBS buffer solution so as to wash off the remaining hydrogen peroxide solution; then, the hydroxylated hollow fullerene aqueous solution of a certain concentration and the cell culture medium were added; after bone marrow cells of mice were incubated for 3 hours, the culture medium containing the hydroxylated hollow fullerene aqueous solution was sucked out, and washing was conducted for three times with PBS buffer solution; finally, the cell culture medium was added, and incubation was continued for 24 hours in a cell incubator; then, cell activities were detected using CCK-8.

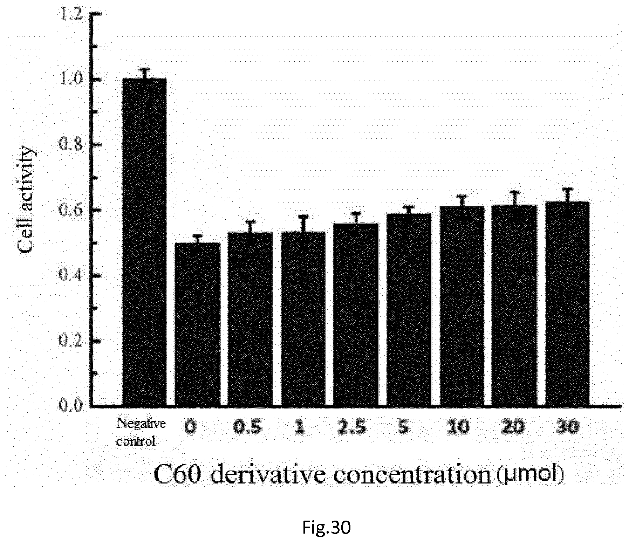

Corresponding detection results are as shown in Figure 30. As can be learned from Figure 30, when 20 µL of hydrogen peroxide solution having a concentration of 100 µM is added, hydrogen peroxide exerts certain killing effects on bone marrow cells of mice, while cell activities increase as the addition amount of the hydroxylated hollow fullerene aqueous solution is increased gradually. This indicates that the hydroxylated hollow fullerene aqueous solution is effective for scavenging free radicals, and is able to protect cells from its influence on a cellular level. Meanwhile, as for the positive control group added merely with the hydroxylated hollow fullerene aqueous solution, but without hydrogen peroxide, its cell activities are slightly higher than those of the negative control group, which indicates that the material herein is not cytotoxic.

### Embodiment 19 Protection effects of water-soluble hydroxylated hollow fullerene on myelosuppression on an intravital level

Animal model: ICR mice aged 4-5 weeks were selected and assigned randomly into 4 groups with 6 mice per group. These groups were, respectively, a blank control group, a water-soluble hydroxylated hollow fullerene experimental group, an X-ray irradiation experimental group and an X-ray+water-soluble hydroxylated hollow fullerene experimental group. 10⁶ mouse hepatocarcinoma cells (H22 cells) were inoculated into right thighs of mice, and the experiment was conducted 5-7 days after the inoculation when the sizes of tumors reached about 5 mm.

Blank control group: The drugs injected intragastrically or intraperitoneally in the experimental groups were replaced with equal volumes of normal saline.

Water-soluble hydroxylated hollow fullerene experimental group: Mice were injected with a water-soluble hydroxylated hollow fullerene aqueous solution (1 mM) via caudal veins at a dosage of 0.004 mmol of water-soluble hydroxylated hollow fullerene/kg of body weight of mice.

X-ray irradiation experimental group: Mice were treated with X-ray whole body radiation in one time at an irradiation dosage of 6Gy.

X-ray+water-soluble hydroxylated hollow fullerene experimental group: Mice were treated with X-ray whole body radiation in one time at an irradiation dosage of 6Gy, and a water-soluble hydroxylated hollow fullerene solution was injected intravenously at a dosage of 0.004 mmol of water-soluble hydroxylated hollow fullerene/kg of body weight of mice.

Injection of drugs was initiated on the 7th day after tumor inoculation, which was marked as the first day of the experiment. Intravenous injection of water-soluble hydroxylated hollow fullerene was conducted once a day for 5 consecutive days; thereafter, blood (20 µl) was taken respectively from orbits of mice on the 4th day, the 7th day, the 10th day, the 14th day and the 17th day, and was placed in a 3 ml centrifuge tube where blood routine examination was conducted using a blood cell automatic analyzer, wherein the main indexes related to myelosuppression are white blood cell counts (WBC), platelet counts (PLT), hemoglobin determination (HGB) and monocyte proportion (MO%).

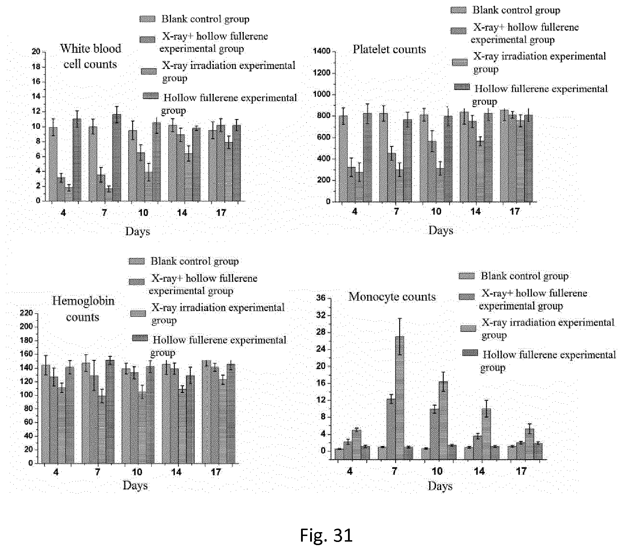

Corresponding detection results are as shown in Figure 31. It can be learned from Figure 31 that as compared with the blank control group, the X-ray experimental group exhibits the following effects: the myelosuppression-related indexes of mice, namely white blood cells, platelets and hemoglobin, are subjected to varying degrees of reduction, wherein the reduction in white blood cells is the most remarkable. The phenomenon of abnormal hyperplasia is observed in their monocytes, which indicates that their bone marrow is damaged, moreover, significant abnormalities are also observed in related indexes; however, as for the mice in the X-ray+water-soluble hydroxylated hollow fullerene experimental group, the numbers of their white blood cells, platelets and hemoglobin are greatly increased as compared with those of the X-ray irradiation experimental group owing to protective effects of water-soluble hydroxylated hollow fullerene, and the number of monocytes is closer to that of the blank control group, moreover, as time progresses, related indexes are getting closer to those of normal mice. This indicates that water-soluble hydroxylated hollow fullerene has obviously protective effects on the myelosuppression of mice caused by radiotherapeutic rays.

### Embodiment 20 Protection effects of water-soluble hydroxylated hollow fullerene on myelosuppression on an intravital level

Animal model: ICR mice aged 4-5 weeks were selected and assigned randomly into 4 groups with 6 mice per group. These groups were, respectively, a blank control group, a water-soluble hydroxylated hollow fullerene experimental group, a cyclophosphamide (CTX) experimental group and a CTX+water-soluble hydroxylated hollow fullerene experimental group. 10⁶ mouse hepatocarcinoma cells (H22 cells) were inoculated into right thighs of mice, and the experiment was conducted 5-7 days after the inoculation when the sizes of tumors reached about 5 mm.

Blank control group: The drugs injected intragastrically or intraperitoneally in the experimental groups were replaced with equal volumes of normal saline.

Water-soluble hydroxylated hollow fullerene experimental group: Mice were injected with a water-soluble hydroxylated hollow fullerene aqueous solution (1 mM) via caudal veins at a dosage of 0.004 mmol of water-soluble hydroxylated hollow fullerene/kg of body weight of mice.

Cyclophosphamide (CTX) experimental group: Mice were injected intraperitoneally with a CTX solution at a dosage of 60 mg/kg of body weight of mice.

CTX+water-soluble hydroxylated hollow fullerene experimental group: Mice were injected intraperitoneally with a CTX solution at a dosage of 60 mg/kg of body weight of mice, and a water-soluble hydroxylated hollow fullerene solution was injected intravenously at a dosage of 0.004 mmol of water-soluble hydroxylated hollow fullerene/kg of body weight of mice.

Injection of drugs was initiated on the 7th day after tumor inoculation, which was marked as the first day of the experiment. Intraperitoneal injection of CTX or intravenous injection of water-soluble hydroxylated hollow fullerene was conducted once a day for 5 consecutive days; thereafter, blood (20 µl) was taken respectively from orbits of mice on the 4th day, the 7th day, the 10th day, the 14th day and the 17th day, and was placed in a 3 ml centrifuge tube where blood routine examination was conducted using a blood cell automatic analyzer, wherein the main indexes related to myelosuppression are white blood cell counts (WBC), platelet counts (PLT), hemoglobin determination (HGB) and monocyte proportion (MO%).

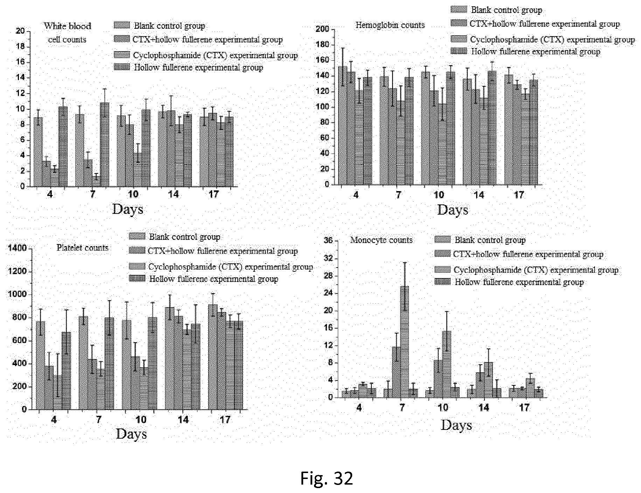

Corresponding detection results are as shown in Figure 32. It can be learned from Figure 32 that as compared with the blank control group, the cyclophosphamide (CTX) experimental group exhibits the following effects: the myelosuppression-related indexes of mice, namely white blood cells, platelets and hemoglobin, are subjected to varying degrees of reduction, wherein the reduction in white blood cells is the most remarkable. The phenomenon of abnormal hyperplasia is observed in their monocytes, which indicates that their bone marrow is damaged, moreover, significant abnormalities are also observed in related indexes; however, as for the mice in the CTX+water-soluble hydroxylated hollow fullerene experimental group, the numbers of their white blood cells, platelets and hemoglobin are greatly increased as compared with those of the cyclophosphamide (CTX) experimental group owing to protective effects of water-soluble hydroxylated hollow fullerene, and the number of monocytes is closer to that of the blank control group, moreover, as time progresses, related indexes are getting closer to those of normal mice. This indicates that water-soluble hydroxylated hollow fullerene has obviously protective effects on the myelosuppression of mice caused by chemotherapeutic drug CTX.

## Claims

**1.** A use of fullerene micro-nano material and/or metallofullerene micro-nano material in the preparation of a medicament, the medicament having at least one of the following properties 1)-7):
1) treatment on myelosuppression; 2) treatment on at least one of myelosuppression-induced leucopenia, platelet reduction, hemoglobin reduction and monocyte reduction; 3) protection on bone marrow cells and/or hematopoietic cells; 4) ability to be enriched in bone marrow; 5) protection on liver tissue, spleen tissue and kidney tissue; 6) scavenging of free radicals; and 7) improvements on the function of an antioxidation system of an organism.

**2.** A method for treating myelosuppression, comprising the step of administering an effective dosage of fullerene micro-nano material and/or metallofullerene micro-nano material into an organism in need of treatment on myelosuppression.

**3.** A pharmaceutical composition for treating myelosuppression, comprising a fullerene micro-nano material and/or metallofullerene micro-nano material and further comprising at least one of pharmaceutically acceptable carrier, pharmaceutically acceptable diluent and pharmaceutically acceptable exipient.

**4.** The use of claim 1, the method of claim 2 or the pharmaceutical composition of claim 3, wherein the fullerene micro-nano material and/or metallofullerene micro-nano material comprises at least one of effective component selected from the group consisting of: an oil-soluble fullerene micro-nano material, an oil-soluble metallofullerene micro-nano material, a composition of the oil-soluble fullerene micro-nano material and the oil-soluble metallofullerene micro-nano material, a water-soluble fullerene micro-nano material, a water-soluble metallofullerene micro-nano material, a composition of the water-soluble fullerene micro-nano material and the water-soluble metallofullerene micro-nano material, pharmaceutically acceptable esters of the above six ingredients or pharmaceutically acceptable salts of the above six ingredients.

**5.** The use of claim 1, the method of claim 2 or the pharmaceutical composition of claim 3, wherein the oil-soluble fullerene micro-nano material is obtained by subjecting a fullerene body material to oil solubility modification, and the oil-soluble metallofullerene micro-nano material is obtained by subjecting a metallofullerene body material to oil solubility modification; the water-soluble fullerene micro-nano material is obtained by subjecting a fullerene body material to water solubility modification, and the water-soluble metallofullerene micro-nano material is obtained by subjecting a metallofullerene body material to water solubility modification.

**6.** The use, method or pharmaceutical composition of claim 5, wherein the fullerene body material comprises one or more cage structures having a general formula of C₂ₘ and composed of carbon atoms, wherein 30≤m≤60.

**7.** The use, method or pharmaceutical composition of claim 5, wherein the metallofullerene body material comprises one or more of M@C₂ₙ, M₂@C₂ₙ, MA@C₂ₙ, M₃N@C₂ₙ, M₂C₂@C₂ₙ, M₂S@C₂ₙ, M₂O@C₂ₙ and MₓA₃₋ₓN@C₂ₙ, both M and A represent metal elements, and are selected from any one of lanthanide metal elements, Sc and Y, and 30≤n≤60, and 0≤x≤3; wherein N represents nitrogen element, C represents carbon element and S represents sulfur element, and lanthanide metal elements comprise La, Ce, Pr, Nd, Pm, Sm, Eu, Gd, Tb, Dy, Ho, Er, Tm, Yb and Lu.

**8.** The use, method or pharmaceutical composition of claim 5, wherein the method for oil solubility modification comprises coating the fullerene body material and/or metallofullerene body material with edible oil.

**9.** The use, method or pharmaceutical composition of claim 8, wherein the edible oil comprises at least one of olive oil, linseed oil, sunflower seed oil, maize germ oil, corn oil and squalane.

**11.** The use, method or pharmaceutical composition of claim 8, wherein the oil-soluble fullerene micro-nano material and/or oil-soluble metallofullerene micro-nano material is gadolinium metallofullerene coated with edible oil, C₆₀ coated with edible oil, C₇₀ coated with edible oil or C₈₄ coated with edible oil.

**12.** The use, method or pharmaceutical composition of claim 5, wherein the method for water solubility modification comprises any one of modifying a carbon cage of the fullerene body material and/or the metallofullerene body material with a hydrophilic group on the outer surface thereof, or having the fullerene body material and/or metallofullerene body material carried by a water-soluble carrier.

**13.** The use, method or pharmaceutical composition of claim 12, wherein the hydrophilic group comprises at least one of a hydroxyl group, an amino group and a carboxy group.

**14.** The use, method or pharmaceutical composition of claim 12, wherein the water-soluble carrier is selected from at least one of lipidosome, polymeric micelle and protein, wherein the polymeric micelle is poly(lactic-co-glycolic acid) polyethylene glycol, polylysine or chitosan, and the protein is albumin or transferrin.

**15.** The use of claim 1, the method of claim 2 or the pharmaceutical composition of claim 3, wherein the myelosuppression is induced by a medical chemotherapy, a chemical toxicant or a medicament having a myelosuppressive side effect.

**16.** The use of claim 15, wherein the medicament employed for the medical chemotherapy is cyclophosphamide, adriamycin, cis-platinum or paclitaxel; the chemical toxicant is benzene or a derivative thereof; and the medicament having a myelosuppressive side effect is chloramphenicol, tetracycline or indometacin.

**17.** The use of claim 1, the method of claim 2 or the pharmaceutical composition of claim 3, wherein the myelosuppression is induced by tumor radiotherapy or rays producing a myelosuppressive side effect.

**18.** The use, method or pharmaceutical composition of claim 17, wherein rays utilized for the radiotherapy are α-rays, β-rays, γ-rays or X-rays.

**19.** The method of claim 2,wherein the fullerene micro-nano material and/or metallofullerene micro-nano material is administered to the organism in need of treatment on myelosuppression during at least one of the following stages: (1) within 24 hours before the administration of the chemotherapeutic drug or the use of radiotherapeutic rays; (2) during the radiotherapy and chemotherapy; (3) within 1-2 weeks after the radiotherapy and chemotherapy are finished.

**20.** The method of claim 2, wherein the effective ingredient is administered at a dosage of from 1 mg/kg/d to 500 mg/kg/d, and alternatively, at a dosage of from 1 mg/kg/d to 100 mg/kg/d, 1 mg/kg/d to 20 mg/kg/d or 1 mg/kg/d to 10 mg/kg/d.

**21.** The method of claim 2, wherein the organism is a human or an animal, and the animal may be a mammal, such as, a mouse, a guinea pig, a rat, a dog, a rabbit and a monkey.
